# EUROPEAN PATENT APPLICATION

(11) **EP 4 806 435 A1**
(43) Date of publication of application: **16.09.2026**
(21) Application number: 24888810.9
(22) Date of filing: 08.11.2024
(51) Int. Cl.: A61K 31/438, A61K 39/395, A61P 35/00, A61P 43/00

(54) **DIHYDROCHROMENE-DERIVATIVE-CONTAINING THERAPEUTIC AGENT FOR CANCER**

(30) Priority: 09.11.2023 US 202363597525 P; 02.10.2024 US 202463702396 P
(71) Applicant: Sumitomo Pharma Co., Ltd., Osaka-shi, Osaka 541-0045 (JP)
(72) Inventor: IINO, Shuichi, Tokyo 103-6012 (JP); SEKI, Masataka, Tokyo 103-6012 (JP); KANAI, Yuki, Osaka-shi, Osaka 541-0045 (JP); EIKAWA, Shingo, Osaka-shi, Osaka 541-0045 (JP); KUSAKABE, Daiki, Osaka-shi, Osaka 554-0022 (JP); YAMASHITA, Akihito, Osaka-shi, Osaka 541-0045 (JP); FURUTA, Yudai, Osaka-shi, Osaka 541-0045 (JP); WATANABE, Tomoko, Osaka-shi, Osaka 541-0045 (JP); LI, Jian, Marlborough, Massachusetts 01752 (US); DOW, Edward, Marlborough, Massachusetts 01752 (US); MEI, JP, Marlborough, Massachusetts 01752 (US); MCMULLEN, Dragana, Marlborough, Massachusetts 01752 (US); XIE, Yuran, Marlborough, Massachusetts 01752 (US)
(74) Representative: Vossius & Partner Patentanwälte Rechtsanwälte mbB
(86) International application number: PCT/JP2024/039748
(87) International publication number: WO 2025/100519

(57) **Abstract**

The present invention relates to preferred administrations, dosages, and uses of a dihydrochromene derivative or a pharmaceutically acceptable salt thereof which is useful as a medicament, as well as a pharmaceutical composition comprising it.

## Description

### TECHNICAL FIELD

The present invention relates to preferred administrations, dosages, and uses of a dihydrochromene derivative or a pharmaceutically acceptable salt thereof which is useful as a medicament, as well as a pharmaceutical composition comprising it.

### BACKGROUND OF THE INVENTION

Recently, Patent Literature 1 reported dihydrochromene derivatives targeting cancer stem cells (hereinafter, sometimes referred to as "CSCs"), which are suggested to play an important role in sustained proliferation of malignant tumors, tumor metastasis, recurrence, and resistance to antitumor agents.

### PRIOR ART

### [Patent Reference]

[Patent Literature 1] WO 2019/181939

### SUMMARY OF THE INVENTION

### (Technical Problem)

The purpose of the present invention is to provide an invention relating to the dosage regimen of a dihydrochromene derivative that exhibits excellent anti-cancer activity by inhibiting emopamil-binding protein (EBP), and to provide a useful therapeutic agent and treatment method for tumors using the derivative and a concomitant drug thereof.

More specifically, the present inventors have found that (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] (hereinafter, also referred to as "the present compound" or "the compound of the present disclosure") or a pharmaceutically acceptable salt thereof inhibits EBP, and have found that it inhibits *de novo* cholesterol biosynthesis and activates liver X receptors (LXRs) through accumulation of zymosterol, a substrate of EBP, thereby depleting intracellular cholesterol and exhibiting cytotoxic activity against cancer cells.

### (Solution to Problem)

The present inventors have extensively studied to reach the above purpose, and then have found that a medicament comprising (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] or a pharmaceutically acceptable salt thereof, or a hydrate or solvate thereof demonstrates excellent anti-cancer activity with a high level of safety when administered at specific dosages and administration conditions. Furthermore, the present inventors have found that the anti-cancer effect can be further enhanced by using a concomitant drug in addition to the above-mentioned medicament. Based upon the findings, the present invention has been achieved.

Accordingly, the present invention is described as follows:
(Item 1) A medicament for treating or preventing a cancer, comprising (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] (hereinafter, also referred to as "the present compound") or a pharmaceutically acceptable salt thereof, or a hydrate or solvate thereof as an active ingredient, which is orally administered to a subject.
(Item 2) The medicament of Item 1, wherein the active ingredient is (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] diphosphate, or a hydrate or solvate thereof.
(Item 3) The medicament of Item 1, wherein the active ingredient is (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] diphosphate (referred to as "Compound 1").
(Item 4) The medicament of any one of Items 1 to 3, which is orally administered to a subject once a day.
(Item 5) The medicament of any one of Items 1 to 3, which is orally administered to a subject twice a day.
(Item 6) The medicament of any one of Items 1 to 5, wherein the single dose of the active ingredient is an amount equivalent to 20 mg of the present compound.
(Item 7) The medicament of any one of Items 1 to 5, wherein the single dose of the active ingredient is an amount equivalent to 40 mg of the present compound.
(Item 8) The medicament of any one of Items 1 to 5, wherein the single dose of the active ingredient is an amount equivalent to 60 mg of the present compound.
(Item 9) The medicament of any one of Items 1 to 5, wherein the single dose of the active ingredient is an amount equivalent to 80 mg of the present compound.
(Item 10) The medicament of any one of Items 1 to 5, wherein the single dose of the active ingredient is an amount equivalent to 100 mg of the present compound.
(Item 11) The medicament of any one of Items 1 to 5, wherein the single dose of the active ingredient is an amount equivalent to 120 mg of the present compound.
(Item 12) The medicament of any one of Items 1 to 5, wherein the single dose of the active ingredient is an amount equivalent to 140 mg of the present compound.
(Item 13) The medicament of any one of Items 1 to 5, wherein the single dose of the active ingredient is an amount equivalent to 160 mg of the present compound.
(Item 14) The medicament of any one of Items 1 to 5, wherein the single dose of the active ingredient is an amount equivalent to 180 mg of the present compound.
(Item 15) The medicament of any one of Items 1 to 5, wherein the single dose of the active ingredient is an amount equivalent to 200 mg of the present compound.
(Item 16) The medicament of any one of Items 1 to 5, wherein the single dose of the active ingredient is an amount equivalent to 220 mg of the present compound.
(Item 17) The medicament of any one of Items 1 to 5, wherein the single dose of the active ingredient is an amount equivalent to 240 mg of the present compound.
(Item 18) The medicament of any one of Items 1 to 5, wherein the single dose of the active ingredient is an amount equivalent to 260 mg of the present compound.
(Item 19) The medicament of any one of Items 1 to 5, wherein the single dose of the active ingredient is an amount equivalent to 280 mg of the present compound.
(Item 20) The medicament of any one of Items 1 to 5, wherein the single dose of the active ingredient is an amount equivalent to 300 mg of the present compound.
(Item 21) The medicament of any one of Items 1 to 5, wherein the single dose of the active ingredient is an amount equivalent to 320 mg of the present compound.
(Item 22) The medicament of any one of Items 1 to 5, wherein the single dose of the active ingredient is an amount equivalent to 340 mg of the present compound.
(Item 23) The medicament of any one of Items 1 to 5, wherein the single dose of the active ingredient is an amount equivalent to 360 mg of the present compound.
(Item 24) The medicament of any one of Items 1 to 5, wherein the single dose of the active ingredient is an amount equivalent to 380 mg of the present compound.
(Item 25) The medicament of any one of Items 1 to 5, wherein the single dose of the active ingredient is an amount equivalent to 400 mg of the present compound.
(Item 26) The medicament of any one of Items 1 to 25, which is used in combination with a concomitant drug or a pharmaceutically acceptable salt thereof to treat a cancer, wherein the concomitant drug is at least one drug selected from an antitumor alkylating agent, an antitumor antimetabolite, an antitumor antibiotic, a plant-derived antitumor medicament, an antitumor platinum complex compound, an antitumor camptothecin derivative, an antitumor tyrosine kinase inhibitor, an antitumor serine/threonine kinase inhibitor, an antitumor phospholipid kinase inhibitor, an antitumor monoclonal antibody, interferon, a biological response modifier, a hormonal preparation, an angiogenic inhibitor, an immune checkpoint inhibitor, an epigenetics-associated molecular inhibitor, a protein post-translational modification inhibitor, a proteasome inhibitor, an enzyme inhibitor, a cholesterol absorption inhibitor, and other antitumor medicaments.
(Item 27) The medicament of any one of Items 1 to 25, which is used in combination with a concomitant drug or a pharmaceutically acceptable salt thereof to treat a cancer, wherein the concomitant drug is at least one selected from an antitumor alkylating agent, an antitumor monoclonal antibody, an immune checkpoint inhibitor, an enzyme inhibitor, a cholesterol absorption inhibitor, and other antitumor medicaments.
(Item 28) The medicament of Item 26 or 27, wherein the immune checkpoint inhibitor is an anti-PD-1 antibody or an anti-PD-L1 antibody.
(Item 29) The medicament of Item 28, wherein the anti-PD-1 antibody or the anti-PD-L1 antibody is pembrolizumab, nivolumab, atezolizumab, durvalumab, avelumab, cemiplimab, dostarlimab, toripalimab, or tislelizumab.
(Item 30) The medicament of Item 26 or 27, wherein the antitumor monoclonal antibody is bevacizumab or ipilimumab.
(Item 31) The medicament of Item 26 or 27, wherein the antitumor alkylating agent is temozolomide.
(Item 32) The medicament of Item 26 or 27, wherein the enzyme inhibitor is an IDH1 inhibitor.
(Item 33) The medicament of Item 32, wherein the IDH1 inhibitor is ivosidenib, vorasidenib, olutasidenib, or enasidenib.
(Item 34) The medicament of Item 26 or 27, wherein the enzyme inhibitor is an IDH1 inhibitor.
(Item 35) The medicament of Item 34, wherein the IDH1 inhibitor is ivosidenib, vorasidenib, or olutasidenib.
(Item 36) The medicament of Item 26 or 27, wherein the cholesterol absorption inhibitor is ezetimibe.
(Item 37) The medicament of any one of Items 1 to 25, which is used in combination with a concomitant drug or a pharmaceutically acceptable salt thereof to treat a cancer, wherein the concomitant drug is at least one selected from
   (1) bevacizumab,
   (2) ipilimumab,
   (3) temozolomide,
   (4) ivosidenib,
   (5) vorasidenib, and
   (6) ezetimibe.
(Item 38) The medicament of any one of Items 1 to 37, which is used in combination with a cell medicine.
(Item 39) The medicament of Item 38, wherein the cell medicine is chimeric antigen receptor T-cell (CAR-T cell).
(Item 40) The medicament of any one of Items 1 to 39, which is used in combination with radiotherapy.
(Item 41) The medicament of any one of Items 1 to 40, wherein the cancer is an anti-PD-1 antibody drug-resistant cancer.
(Item 42) The medicament of any one of Items 1 to 41, wherein the cancer is glioma.
(Item 43) The medicament of Item 42, wherein the glioma is malignant glioma.
(Item 44) The medicament of Item 43, wherein the malignant glioma is recurrent malignant glioma.
(Item 45) The medicament of any one of Items 42 to 44, wherein the glioma is diffuse glioma, pediatric-type diffuse low-grade glioma, pediatric-type diffuse high-grade glioma, circumscribed astrocytic glioma, glioneuronal and neuronal tumor, or ependymal tumor.
(Item 46) The medicament of Item 45, wherein the glioma is diffuse glioma.
(Item 47) The medicament of Item 45 or 46, wherein the diffuse glioma is astrocytoma, oligodendroglioma, or glioblastoma.
(Item 48) The medicament of any one of Items 45 to 47, wherein the diffuse glioma is glioblastoma.
(Item 49) The medicament of Item 47 or 48, wherein the glioblastoma is secondary glioblastoma.
(Item 50) The medicament of Item 45 or 46, wherein the diffuse glioma is astrocytoma or oligodendroglioma.
(Item 51) The medicament of Item 45 or 46, wherein the diffuse glioma is anaplastic oligodendroglioma or anaplastic astrocytoma.
(Item 52) The medicament of Item 45 or 46, wherein the diffuse glioma is pilocytic astrocytoma.
(Item 53) The medicament of Item 42, wherein the glioma is grade I or II based on the WHO 2016 classification of brain tumors.
(Item 54) The medicament of Item 42, wherein the glioma is grade II or III based on the WHO 2016 classification of brain tumors.
(Item 55) The medicament of Item 42, wherein the glioma is grade III or IV based on the WHO 2016 classification of brain tumors.
(Item 56) The medicament of Item 42, wherein the glioma is grade I to III based on the WHO 2016 classification of brain tumors.
(Item 57) The medicament of any one of Items 1 to 56, wherein the cancer is a cancer in which emopamil binding protein is highly expressed compared to that of healthy individuals.
(Item 58) The medicament of any one of Items 1 to 57, wherein the cancer is a cancer with IDH mutation.
(Item 59) The medicament of any one of Items 1 to 40, which is administered to a subject having IDH mutation.
(Item 60) The medicament of Item 59, wherein the subject having IDH mutation is determined through the following steps:
   (1) detecting IDH mutation in cancer cells obtained from the subject, and
   (2) determining the presence or absence of the IDH mutation detected in (1).

### (Item 1A)

A method for treating and/or preventing a cancer, comprising orally administering a therapeutically effective amount of (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] (referred to as "the present compound") or a pharmaceutically acceptable salt thereof, or a hydrate or solvate thereof as an active ingredient to a subject in need thereof.

### (Item 2A)

The method of Item 1A, wherein the active ingredient is (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] diphosphate, or a hydrate or solvate thereof.

### (Item 3A)

The method of Item 1A, wherein the active ingredient is (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] diphosphate (referred to as "Compound 1").

### (Item 4A)

The method of any one of Items 1A to 3A, wherein the active ingredient is administered once a day.

### (Item 5A)

The method of any one of Items 1A to 3A, wherein the active ingredient is administered twice a day.

### (Item 6A)

The method of any one of Items 1A to 5A, wherein the single dose of the active ingredient is an amount equivalent to 20 mg of the present compound.

### (Item 7A)

The method of any one of Items 1A to 5A, wherein the single dose of the active ingredient is an amount equivalent to 40 mg of the present compound.

### (Item 8A)

The method of any one of Items 1A to 5A, wherein the single dose of the active ingredient is an amount equivalent to 60 mg of the present compound.

### (Item 9A)

The method of any one of Items 1A to 5A, wherein the single dose of the active ingredient is an amount equivalent to 80 mg of the present compound.

### (Item 10A)

The method of any one of Items 1A to 5A, wherein the single dose of the active ingredient is an amount equivalent to 100 mg of the present compound.

### (Item 11A)

The method of any one of Items 1A to 5A, wherein the single dose of the active ingredient is an amount equivalent to 120 mg of the present compound.

### (Item 12A)

The method of any one of Items 1A to 5A, wherein the single dose of the active ingredient is an amount equivalent to 140 mg of the present compound.

### (Item 13A)

The method of any one of Items 1A to 5A, wherein the single dose of the active ingredient is an amount equivalent to 160 mg of the present compound.

### (Item 14A)

The method of any one of Items 1A to 5A, wherein the single dose of the active ingredient is an amount equivalent to 180 mg of the present compound.

### (Item 15A)

The method of any one of Items 1A to 5A, wherein the single dose of the active ingredient is an amount equivalent to 200 mg of the present compound.

### (Item 16A)

The method of any one of Items 1A to 5A, wherein the single dose of the active ingredient is an amount equivalent to 220 mg of the present compound.

### (Item 17A)

The method of any one of Items 1A to 5A, wherein the single dose of the active ingredient is an amount equivalent to 240 mg of the present compound.

### (Item 18A)

The method of any one of Items 1A to 5A, wherein the single dose of the active ingredient is an amount equivalent to 260 mg of the present compound.

### (Item 19A)

The method of any one of Items 1A to 5A, wherein the single dose of the active ingredient is an amount equivalent to 280 mg of the present compound.

### (Item 20A)

The method of any one of Items 1A to 5A, wherein the single dose of the active ingredient is an amount equivalent to 300 mg of the present compound.

### (Item 21A)

The method of any one of Items 1A to 5A, wherein the single dose of the active ingredient is an amount equivalent to 320 mg of the present compound.

### (Item 22A)

The method of any one of Items 1A to 5A, wherein one dose of the active ingredient is 340 mg converted to the present compound.

### (Item 23A)

The method of any one of Items 1A to 5A, wherein the single dose of the active ingredient is an amount equivalent to 360 mg of the present compound.

### (Item 24A)

The method of any one of Items 1A to 5A, wherein the single dose of the active ingredient is an amount equivalent to 380 mg of the present compound.

### (Item 25A)

The method of any one of Items 1A to 5A, wherein the single dose of the active ingredient is an amount equivalent to 400 mg of the present compound.

### (Item 26A)

The method of any one of Items 1A to 25A for treating a cancer, wherein the active ingredient is administered in combination with a concomitant drug or a pharmaceutically acceptable salt thereof, wherein the concomitant drug is at least one drug selected from an antitumor alkylating agent, an antitumor antimetabolite, an antitumor antibiotic, a plant-derived antitumor medicament, an antitumor platinum complex compound, an antitumor camptothecin derivative, an antitumor tyrosine kinase inhibitor, an antitumor serine/threonine kinase inhibitor, an antitumor phospholipid kinase inhibitor, an antitumor monoclonal antibody, interferon, a biological response modifier, a hormonal preparation, an angiogenic inhibitor, an immune checkpoint inhibitor, an epigenetics-associated molecular inhibitor, a protein post-translational modification inhibitor, a proteasome inhibitor, an enzyme inhibitor, a cholesterol absorption inhibitor, and other antitumor medicaments.

### (Item 27A)

The method of any one of Items 1A to 25A for treating a cancer, wherein the active ingredient is administered in combination with a concomitant drug or a pharmaceutically acceptable salt thereof, wherein the concomitant drug is at least one drug selected from an antitumor alkylating agent, an antitumor monoclonal antibody, an immune checkpoint inhibitor, an enzyme inhibitor, a cholesterol absorption inhibitor, and other antitumor medicaments.

### (Item 28A)

The method of Item 26A or 27A, wherein the immune checkpoint inhibitor is an anti-PD-1 antibody or an anti-PD-L1 antibody.

### (Item 29A)

The method of Item 28A, wherein the anti-PD-1 antibody or the anti-PD-L1 antibody is pembrolizumab, nivolumab, atezolizumab, durvalumab, avelumab, cemiplimab, dostarlimab, toripalimab, or tislelizumab.

### (Item 30A)

The method of Item 26A or 27A, wherein the antitumor monoclonal antibody is bevacizumab or ipilimumab.

### (Item 31A)

The method of Item 26A or 27A, wherein the antitumor alkylating agent is temozolomide.

### (Item 32A)

The method of Item 26A or 27A, wherein the enzyme inhibitor is an IDH inhibitor.

### (Item 33A)

The method of Item 32A, wherein the IDH inhibitor is ivosidenib, vorasidenib, olutasidenib, or enasidenib.

### (Item 34A)

The method of Item 26A or 27A, wherein the enzyme inhibitor is an IDH1 inhibitor.

### (Item 35A)

The method of Item 34A, wherein the IDH1 inhibitor is ivosidenib, vorasidenib, or olutasidenib.

### (Item 36A)

The method of Item 26A or 27A, wherein the cholesterol absorption inhibitor is ezetimibe.

### (Item 37A)

The method of any one of Items 1A to 25A for treating a cancer, wherein the active ingredient is administered in combination with a concomitant drug or a pharmaceutically acceptable salt thereof, wherein the concomitant drug is at least one drug selected from
(1) bevacizumab,
(2) ipilimumab,
(3) temozolomide,
(4) ivosidenib,
(5) vorasidenib, and
(6) ezetimibe.

### (Item 38A)

The method of any one of Items 1A to 37A, which is done in combination with the administration of a cell medicine.

### (Item 39A)

The method of Item 38A, wherein the cell medicine is chimeric antigen receptor T cell (CAR-T cell).

### (Item 40A)

The method of any one of Items 1A to 39A, which is done in combination with radiotherapy.

### (Item 41A)

The method of any one of Items 1A to 40A, wherein the cancer is an anti-PD-1 antibody drug-resistant cancer.

### (Item 42A)

The method of any one of Items 1A to 41A, wherein the cancer is glioma.

### (Item 43A)

The method of Item 42A, wherein the glioma is malignant glioma.

### (Item 44A)

The method of Item 43A, wherein the malignant glioma is recurrent malignant glioma.

### (Item 45A)

The method of any one of Items 42A to 44A, wherein the glioma is diffuse glioma, pediatric-type diffuse low-grade glioma, pediatric-type diffuse high-grade glioma, circumscribed astrocytic glioma, glial neuronal tumor and neuronal tumor, or ependymal tumor.

### (Item 46A)

The method of Item 45A, wherein the glioma is diffuse glioma.

### (Item 47A)

The method of Item 45A or 46A, wherein the diffuse glioma is astrocytoma, oligodendroglioma, or glioblastoma.

### (Item 48A)

The method of any one of Items 45A to 47A, wherein the diffuse glioma is glioblastoma.

### (Item 49A)

The method of Item 47A or 48A, wherein the glioblastoma is secondary glioblastoma.

### (Item 50A)

The method of Item 45A or 46A, wherein the diffuse glioma is astrocytoma or oligodendroglioma.

### (Item 51A)

The method of Item 45A or 46A, wherein the diffuse glioma is anaplastic oligodendroglioma or anaplastic astrocytoma.

### (Item 52A)

The method of Item 45A or 46A, wherein the diffuse glioma is pilocytic astrocytoma.

### (Item 53A)

The method of Item 42A, wherein the glioma is grade I or II based on the WHO 2016 classification of brain tumors.

### (Item 54A)

The method of Item 42A, wherein the glioma is grade II or III based on the WHO 2016 classification of brain tumors.

### (Item 55A)

The method of Item 42A, wherein the glioma is grade III or IV based on the WHO 2016 classification of brain tumors.

### (Item 56A)

The method of Item 42A, wherein the glioma is grade I - III based on the WHO 2016 classification of brain tumors.

### (Item 57A)

The method of any one of Items 1A to 56A, wherein the cancer is a cancer in which emopamil binding protein is highly expressed compared to that of healthy individuals.

### (Item 58A)

The method of any one of Items 1A to 57A, wherein the cancer is a cancer with IDH mutation.

### (Item 59A)

The method of any one of Items 1A to 40A, wherein the subject has IDH mutation.

### (Item 60A)

The method of Item 59A, wherein the subject having IDH mutation is determined through the following steps:
(1) detecting IDH mutation in cancer cells obtained from the subject, and
(2) determining the presence or absence of the IDH mutation detected in (1).

### (Effect of the Invention)

The technology of the present disclosure is useful as a therapeutic and/or prophylactic agent for glioma, wherein the compound of the present invention or a pharmaceutically acceptable salt, or hydrate or solvate thereof is administered in accordance with the dosage and administration described herein, and exhibits excellent anticancer activity against cancer cells. Without being limited thereto, the technology of the present disclosure can be used as various therapeutic and/or prophylactic agents by administering to a human at a dose that has been confirmed or is expected to be well tolerated. Furthermore, the compound of the present invention or a pharmaceutically acceptable salt, or hydrate or solvate thereof may be used in combination with one or more concomitant drugs to further enhance the anticancer activity.

The purpose of the present disclosure is to provide an invention relating to the dosage and administration of a dihydrochromene derivative that exhibits excellent anticancer activity by inhibiting binding to emopamil-binding protein (EBP), and to further provide a useful therapeutic agent and a method for treating tumors by combination with other anticancer agents.

More specifically, the present inventors provide a technology related to a medicine comprising (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] or a pharmaceutically acceptable salt thereof, or a hydrate or solvate thereof.

### DESCRIPTION OF THE DRAWINGS

Fig. 1 shows the study design of a clinical trial using Compound 1 in patients with recurrent malignant glioma classified as WHO grade III or IV.
Fig. 2 shows the patient selection criteria in a clinical trial using Compound 1 in patients with recurrent malignant glioma classified as WHO grade III or IV (Baseline Characteristics).
Fig. 3 shows the results of dose-limiting toxicities in a clinical trial (dose-escalation study part) using Compound 1 in patients with recurrent malignant glioma classified as WHO grade III or IV (Data Cut-off: 2023/09/22).
Fig. 4 shows safety data in a clinical trial (dose-escalation study part) using Compound 1 in patients with recurrent malignant glioma classified as WHO grade III or IV (Data Cut-off: 2023/09/22).
Fig. 5 shows Lathosterol/Zymostenol (L/Z) ratio in a clinical trial (dose-escalation study part) using Compound 1 in patients with recurrent malignant glioma classified as WHO grade III or IV (Data Cut-off: September 2023).
Fig. 6 shows relationship between treatment duration and treatment efficacy in a clinical trial (dose-escalation study part) using Compound 1 in patients with recurrent malignant glioma classified as WHO grade III or IV (Data Cut-off: 2023/09/22).
Fig. 7 shows preliminary antitumor effect in a clinical trial (dose-escalation study part) using Compound 1 in patients with recurrent malignant glioma classified as WHO grade III or IV (Data Cut-off: 2023/09/11).
Fig. 8 shows brain MRI images of an anaplastic astrocytoma patient enrolled in the 120 mg QD cohort who achieved a complete response, in a clinical trial (dose-escalation study part) using Compound 1 in patients with recurrent malignant glioma classified as WHO grade III or IV.
Fig. 9 shows shows preliminary antitumor effect in a clinical trial (dose-escalation study part) using Compound 1 in patients with recurrent malignant glioma classified as WHO grade III or IV (Data Cut-off: 2024/04/22).
Fig. 10 shows Lathosterol/Zymostenol (L/Z) ratio in a clinical trial (dose-escalation study part) using Compound 1 in patients with recurrent malignant glioma classified as WHO grade III or IV (as of April 2024).
Fig. 11 shows the mean plasma concentration-time curves of Compound 1 in the 20 mg to 240 mg once-daily dose groups in a clinical study in patients with recurrent malignant glioma of WHO grade III or IV (data cut-off: April 2023).
Fig. 12 shows the mean plasma concentration-time curves of Compound 1 in the 20 mg once-daily to 240 mg twice-daily dose groups in a clinical study in patients with recurrent malignant glioma of WHO grade III or IV (data cut-off: March 2024).
Fig. 13 shows the blood L/Z ratio relative to the blood exposure of Compound 1 in each dose group in a clinical study (dose-escalation part) using Compound 1 in patients with recurrent malignant glioma of WHO grade III or IV.
Fig. 14 shows the study design of a clinical trial in patients with recurrent malignant glioma of WHO grade II, III, or IV.
Fig. 15 shows the antitumor effect in a combination efficacy evaluation of Compound 1 and bevacizumab in mice bearing luciferase-expressing U-87 cells.
Fig. 16 shows the antitumor effect in a combination efficacy evaluation of Compound 1 and ipilimumab in mice bearing MC38 cells.
Fig. 17 shows the antitumor effect in a combination efficacy evaluation of Compound 1 and ipilimumab in mice bearing B16 cells.
Fig. 18 shows the antitumor effect of Compound 1 as a monotherapy in an efficacy evaluation using mice bearing B16 cells which are anti-PD-1 antibody-resistant cancer model.
Fig. 19 shows the cytotoxicity of Compound 1 in the cytotoxicity assay using IDH1 R132H mutant U-87 cells under *in vitro* sphere culture conditions with low-density lipoprotein (LDL) supplementation.
Fig. 20 shows the results of evaluating the cytotoxicity of Compound 1 in combination with ezetimibe in U-87 cells (IDH1 wild-type) under *in vitro* sphere culture conditions with low-density lipoprotein (LDL) supplementation.
Fig. 21 shows the predicted metabolic pathways of the present compound.
Fig. 22 shows the results of evaluating the cytotoxicity under *in vitro* sphere culture conditions using the metabolites.
Fig. 23 shows the results of evaluating the cytotoxicity of Compound 1 under sphere culture conditions with the addition of low-density lipoprotein (LDL), using the low-grade glioma-derived cell line BT142.

### DETAILED DESCRIPTION OF THE PREFERRED EMBODIMENTS

It should be understood that throughout the present specification, expressions in the singular also include the concept of the plural, unless otherwise indicated. Thus, articles in the singular (e.g. "a", "an", "the", etc. in the English language) should be understood to include their plural forms as well, unless otherwise indicated. Also, it should be understood that the terms used in the present specification have the meanings commonly used in the relevant field, unless otherwise indicated. Thus, unless otherwise defined, all technical and scientific terms used herein have the same meaning as commonly understood by a skilled person in the art to which the present disclosure belongs. In case of conflict, the present specification (including definitions) will control.

(4'aR,10'bR)-8'-Chloro-1-[2-(1H-imidazol-1-yl)ethyl]-5',5'-dimethyl-4'a,10'b-dihydro-2'H,4'H,5'H-spiro[piperidine-4,3'-pyrano[3,2-c] [1]benzopyran] diphosphate (Compound 1) has the following structure:

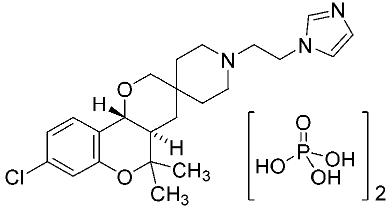

In the phrase "the present compound or a pharmaceutically acceptable salt thereof, or a hydrate or solvate thereof", the term "a hydrate or solvate thereof" is intended to mean a hydrate or solvate of the present compound, as well as a hydrate or solvate of a pharmaceutically acceptable salt of the present compound.

The present compound means (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] and refers to the free (non-salt) form of the compound.

Compound 1 means (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] diphosphate.

The "pharmaceutically acceptable salt" includes acid addition salts and base addition salts. For example, the acid addition salt includes inorganic acid salts such as hydrochloride, hydrobromide, sulfate, hydroiodide, nitrate, and phosphate; or organic acid salts such as citrate, oxalate, phthalate, fumarate, maleate, succinate, malate, acetate, formate, propionate, benzoate, trifluoroacetate, methanesulfonate, benzenesulfonate, p-toluenesulfonate, and camphorsulfonate. The base addition salt includes inorganic base salts such as sodium salts, potassium salts, calcium salts, magnesium salts, barium salts, and aluminum salts; and organic base salts such as trimethylamine, triethylamine, pyridine, picoline, 2,6-lutidine, ethanolamine, diethanolamine, triethanolamine, tromethamine[tris(hydroxymethyl)methylamine], tert-butylamine, cyclohexylamine, dicyclohexylamine, and N,N-dibenzylethylamine. The "pharmaceutically acceptable salt" also includes amino acid salts of basic or acidic amino acids such as arginine, lysine, ornithine, aspartate, and glutamate.

The compound provided herein can encompass a variety of stereochemical forms. The compound of the present invention encompasses not only optical isomers but also diastereomers, for example, a mixture of enantiomers which includes a racemic mixture, and individual enantiomers and diastereomers that result from structural asymmetry in a particular compound. Separation of individual isomers or selective synthesis of individual isomers is achieved by applying various methods known to those skilled in the art.

The present compound or a pharmaceutically acceptable salt thereof, or a hydrate or solvate thereof includes various hydrates, solvates, and crystal polymorphs. Furthermore, the compound of the present invention may be substituted with an isotopic element (e.g., ²H (or D), ³H (or T), ¹¹C, ¹³C, ¹⁴C, ¹³N, ¹⁵N, ¹⁵O, ³⁵S, ¹⁸F, ¹²⁵I, etc.). These compounds are also included in the compound of the present invention.

In addition, the scope disclosed herein includes a prodrug of the present compound. In the present disclosure, a prodrug refers to a derivative that is decomposed *in vivo* by acid hydrolysis or enzymatically to yield the present compound. For example, a prodrug can be produced by modifying the amino group of the present compound according to a conventional method. Specifically, it includes a compound in which the amino group is substituted with an alkanoyl group to become an alkanoylamino group, a compound in which the amino group is substituted with an alkoxycarbonyl group to become an alkoxycarbonylamino group, a compound in which the amino group is transformed to an alkanoyloxymethylamino group, and a compound in which the amino group is transformed to a hydroxylamine.

Hereinafter, terms used herein are explained as follows.

In some embodiments, the subject treated by the above methods is a mammal. The present compound can be used to mammals, and the term "mammal" is used herein in its normal biological meaning. Accordingly, it includes, for example, humans, cows, horses, dogs, cats, rats, and mice, but also many other species. In some further embodiments, the subject is a human.

If it is desirable to obtain the compound of the present invention as a pharmaceutically acceptable salt, when the present compound is obtained as a pharmaceutically acceptable salt, it may be purified without further reaction, and when it is obtained in a free form, it may be dissolved or suspended in an appropriate organic solvent and an acid or base may be added therein to form a salt in a general manner.

In the present invention, the "drug used in combination" or "concomitant drug" is an antitumor medicament which can be used in combination with the compound of the present invention or a pharmaceutically acceptable salt thereof, or a hydrate or solvate thereof, or can be combined with the compound of the present invention or a pharmaceutically acceptable salt thereof, or a hydrate or solvate thereof in a pharmaceutical composition. The "drug used in combination (concomitant drug)" includes, for example, an antitumor alkylating agent, an antitumor antimetabolite, antitumor antibiotics, a plant-derived antitumor agent, an antitumor platinum coordination compound, an antitumor camptothecin derivative, an antitumor tyrosine kinase inhibitor, an antitumor serine/threonine kinase inhibitor, an antitumor phospholipid kinase inhibitor, an antitumor monoclonal antibody, interferon , a biological response modifier, a hormonal agent, an angiogenesis inhibitor, an immune checkpoint inhibitor, an epigenetics-related molecular inhibitor, a post-translational protein modification inhibitor, a proteasome inhibitor, an enzyme inhibitor, a cholesterol absorption inhibitor, and other antitumor agents. Examples of the "drug used in combination (concomitant drug)" include, for example, azacytidine, vorinostat, decitabine, romidepsin, idarubicin, daunorubicin, doxorubicin, enocitabine, cytarabine, mitoxantrone, thioguanine, etoposide, ifosfamide, cyclophosphamide, dacarbazine, temozolomide, nimustine, busulfan, procarbazine, melphalan, ranimustine, all-trans-retinoic acid, tamibarotene, cisplatin, carboplatin, oxaliplatin, irinotecan, bleomycin, mitomycin C, methotrexate, paclitaxel, docetaxel, gemcitabine, tamoxifen, thiotepa, tegafur, fluorouracil, everolimus, temsirolimus, gefitinib, erlotinib, imatinib, crizotinib, osimertinib, afatinib, dasatinib, bosutinib, vandetanib, sunitinib, axitinib, pazopanib, lenvatinib, lapatinib, nilotinib, ibrutinib, ceritinib, alectinib, tofacitinib, baricitinib, ruxolitinib, olaparib, sorafenib, vemurafenib, dabrafenib, trametinib, palbociclib, bortezomib, carfilzomib, rituximab, cetuximab, trastuzumab, bevacizumab, panitumumab, nivolumab, atezolizumab, mogamulizumab, alemtuzumab, ofatumumab, ipilimumab, ramucirumab, brentuximab vedotin, gemtuzumab ozogamicin, inotuzumab ozogamicin, venetoclax, gilteritinib, vorasidenib, ivosidenib, olutasidenib, enasidenib, pembrolizumab, durvalumab, avelumab, cemiplimab, dostarlimab, toripalimab, tislelizumab, ezetimibe, and the like.
The cell medicine is a drug that exerts a therapeutic effect when administered as cells themselves, regardless of the origin of the cells or the degree of processing involved. Examples include chimeric antigen receptor T cells (CAR-T cells), TCR-T cells, and NK cells. Preferably, the cell medicine is a chimeric antigen receptor T cell (CAR-T cell). Specific examples include idecabtagene vicleucel, lisocabtagene maraleucel, ciltacabtagene autoleucel, tisagenlecleucel, and axicabtagene ciloleucel.

Radiation therapy is a treatment method in which high-energy X-rays or other forms of radiation are delivered to the affected area from outside or inside the body to damage tumor cells.

"U-87 cells" refer to a human malignant glioma cell line derived from epithelial tissue, and have characteristics similar to those of glioblastoma. For the purpose of observing tumor growth within brain tissue, luciferase-expressing U-87 cells were established by forcibly expressing luciferase, a bioluminescent enzyme, in the cells.

"MC38 cells" refer to a methylcholanthrene-induced colon adenocarcinoma cell line derived from C57BL/6 mice, and are considered to be relatively highly immunogenic.

"B16 cells" refer to a malignant melanoma cell line that arose spontaneously in C57BL/6 mice.

Glioma includes, for example, diffuse glioma, pediatric-type diffuse low-grade glioma, pediatric-type diffuse high-grade glioma, circumscribed astrocytic glioma, glioneuronal and neuronal tumors, and ependymal tumor. Diffuse glioma includes, for example, astrocytoma with IDH mutation, oligodendroglioma with IDH mutation and 1p/19q co-deletion, and glioblastoma. With regard to the classification of gliomas, Volume 6 of the WHO Classification of Tumours, 5th Edition, was referenced.

Gliomas are classified into Grades I to IV based on the WHO 2016 classification (revised 4th edition), which employs diagnostic criteria combining morphological and molecular diagnoses.

Glioblastoma comprises two subsets: secondary glioblastoma which arises through malignant transformation from a benign astrocytoma, and de novo glioblastoma which develops in the absence of a precursor lesion.

Malignant glioma refers to a glioma characterized by rapid growth, infiltration into adjacent normal tissue, and poorly defined borders. According to the WHO classification determined by pathological examination, it corresponds to gliomas classified as Grade III or Grade IV among the four grading tiers. In contrast, low grade glioma refers to gliomas classified as Grade I or Grade II. For example, oligodendroglioma is classified as Grade II, whereas a tumor that has undergone malignant transformation to Grade III is referred to as anaplastic oligodendroglioma. Astrocytoma is classified as a Grade II tumor in terms of malignancy, whereas a tumor that has undergone malignant transformation to Grade III is referred to as anaplastic astrocytoma. In addition, pilocytic astrocytoma is classified as Grade I.

Mutations in IDH1 or IDH2 have been reported to occur predominantly in the majority of Grade II or Grade III gliomas based on the WHO 2016 classification (revised 4th edition), as well as in secondary glioblastomas arising from these precursor lesions (N. Engl. J. Med. 360, 765-773 (2009)).

Recurrence refers to the reappearance of cancer that had previously been confirmed to have apparently disappeared as a result of treatment. Recurrence includes not only cases in which the cancer reappears at or near the site of the original primary lesion, but also cases in which it is found in a different organ as a "metastasis."

The frequency of oral administration of the medicament comprising the present compound or a pharmaceutically acceptable salt thereof, or a hydrate or solvate thereof, may be once a day, twice a day, three times a day, or four times a day. Preferably, it is administered once a day or twice a day, more preferably once a day. In another preferred embodiment, it is administered twice a day.

The term "an amount equivalent to the present compound" means that the administered dose contains the same molar amount of the present compound.

For example, the administered dose of Compound 1 is calculated using the following formula: 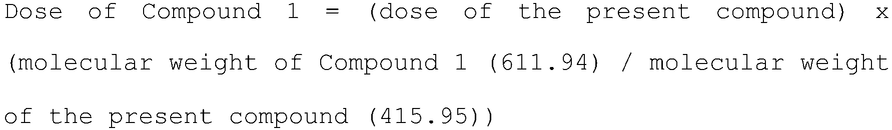 Dose of Compound 1 = (dose of the present compound) x (molecular weight of Compound 1 (611.94) / molecular weight of the present compound (415.95))

Specifically, Compound 1 of "an amount equivalent to 20 mg of the present compound" means: 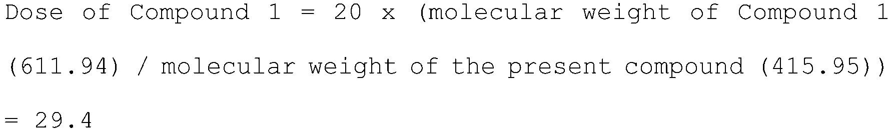 Dose of Compound 1 = 20 x (molecular weight of Compound 1 (611.94) / molecular weight of the present compound (415.95)) = 29.4 Thus, this corresponds to 29.4 mg of Compound 1.

The same calculation can be applied when the compound is in the form of any other salt, hydrate, solvate, or similar form.

Single oral dose of the present compound or a pharmaceutically acceptable salt thereof, or a hydrate or solvate thereof in the medicament includes 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg, 510 mg, 520 mg, 530 mg, 540 mg, 550 mg, 560 mg, 570 mg, 580 mg, 590 mg, 600 mg, 610 mg, 620 mg, 630 mg, 640 mg, 650 mg, 660 mg, 670 mg, 680 mg, 690 mg, 700 mg, 710 mg, 720 mg, 730 mg, 740 mg, 750 mg, 760 mg, 770 mg, 780 mg, 790 mg, 800 mg, 810 mg, 820 mg, 830 mg, 840 mg, 850 mg, 860 mg, 870 mg, 880 mg, 890 mg, 900 mg, 910 mg, 920 mg, 930 mg, 940 mg, 950 mg, 960 mg, 970 mg, 980 mg, 990 mg, 1000 mg, 1010 mg, 1020 mg, 1030 mg, 1040 mg, 1050 mg, 1060 mg, 1070 mg, 1080 mg, 1090 mg, 1100 mg, 1110 mg, 1120 mg, 1130 mg, 1140 mg, 1150 mg, 1160 mg, 1170 mg, 1180 mg, 1190 mg, and 1200 mg, each of which is calculated as the present compound (free form). And, it also includes the range between these two arbitrarily-selected doses.

Single oral dose of the present compound or a pharmaceutically acceptable salt thereof, or a hydrate or solvate thereof in the medicament includes, preferably, 20 mg, 40 mg, 60 mg, 80 mg, 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, 400 mg, 420 mg, 440 mg, 460 mg, 480 mg, 500 mg, 520 mg, 540 mg, 560 mg, 580 mg, 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, and 1000 mg, each of which is calculated as the present compound (free form). And, it also includes the range between these two arbitrarily-selected doses.

Single oral dose of the present compound or a pharmaceutically acceptable salt thereof, or a hydrate or solvate thereof in the medicament includes, more preferably, 20 mg, 40 mg, 60 mg, 80 mg, 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, 400 mg, 420 mg, 440 mg, 460 mg, 480 mg, 500 mg, 520 mg, 540 mg, 560 mg, 580 mg, and 600 mg, each of which is calculated as the present compound (free form). And, it also includes the range between these two arbitrarily-selected doses.

Single oral dose of the present compound or a pharmaceutically acceptable salt thereof, or a hydrate or solvate thereof in the medicament includes, even more preferably, 20 mg, 40 mg, 60 mg, 80 mg, 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, 400 mg, 420 mg, 440 mg, 460 mg, 480 mg, and 500 mg, each of which is calculated as the present compound (free form). And, it also includes the range between these two arbitrarily-selected doses.

Single oral dose of the present compound or a pharmaceutically acceptable salt thereof, or a hydrate or solvate thereof in the medicament includes, the most preferably, 20 mg, 40 mg, 60 mg, 80 mg, 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, and 400 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 20 mg, 40 mg, 60 mg, 80 mg, 100 mg, 120 mg, 140 mg, 160 mg, 180 mg, 200 mg, 220 mg, 240 mg, 260 mg, 280 mg, 300 mg, 320 mg, 340 mg, 360 mg, and 380 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 20 mg, 40 mg, 60 mg, 80 mg, 100 mg, 120 mg, 140 mg, 160 mg, 180 mg, 200 mg, 220 mg, 240 mg, 260 mg, 280 mg, 300 mg, 320 mg, 340 mg, and 360 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 20 mg, 40 mg, 60 mg, 80 mg, 120 mg, 180 mg, 240 mg, and 360 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 120 mg, 180 mg, 240 mg, and 360 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 20 mg, 40 mg, 60 mg, and 80 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 120 mg, 140 mg, 160 mg, 180 mg, 200 mg, 220 mg, 240 mg, 260 mg, and 280 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 120 mg, 140 mg, 160 mg, 180 mg, 200 mg, 220 mg, 240 mg, and 260 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 140 mg, 160 mg, 180 mg, 200 mg, 220 mg, 240 mg, and 260 mg, each of which is calculated as the present compound (free form). And, it also includes the range between these two arbitrarily-selected doses.

In another preferred embodiment, single oral dose of the present compound or a pharmaceutically acceptable salt thereof, or a hydrate or solvate thereof in the medicament includes 140 mg, 160 mg, 180 mg, 200 mg, 220 mg, and 240 mg, each of which is calculated as the present compound (free form). And, it also includes the range between these two arbitrarily-selected doses.

In another more preferred embodiment, single oral dose of the present compound or a pharmaceutically acceptable salt thereof, or a hydrate or solvate thereof in the medicament includes 160 mg, 180 mg, 200 mg, 220 mg, and 240 mg, each of which is calculated as the present compound (free form). And, it also includes the range between these two arbitrarily-selected doses.

In another further more preferred embodiment, single oral dose of the present compound or a pharmaceutically acceptable salt thereof, or a hydrate or solvate thereof in the medicament includes 160 mg, 180 mg, 200 mg, and 220 mg, each of which is calculated as the present compound (free form). And, it also includes the range between these two arbitrarily-selected doses.

In another most preferred embodiment, single oral dose of the present compound or a pharmaceutically acceptable salt thereof, or a hydrate or solvate thereof in the medicament includes 160 mg, 180 mg, and 200 mg, each of which is calculated as the present compound (free form). And, it also includes the range between these two arbitrarily-selected doses.

In another most preferred embodiment, single oral dose of the present compound or a pharmaceutically acceptable salt thereof, or a hydrate or solvate thereof in the medicament includes 20 mg which is calculated as the present compound (free form).

In another most preferred embodiment, single oral dose of the present compound or a pharmaceutically acceptable salt thereof, or a hydrate or solvate thereof in the medicament includes 40 mg which is calculated as the present compound (free form).

In another most preferred embodiment, single oral dose of the present compound or a pharmaceutically acceptable salt thereof, or a hydrate or solvate thereof in the medicament includes 60 mg which is calculated as the present compound (free form).

In another most preferred embodiment, single oral dose of the present compound or a pharmaceutically acceptable salt thereof, or a hydrate or solvate thereof in the medicament includes 80 mg which is calculated as the present compound (free form).

In another most preferred embodiment, single oral dose of the present compound or a pharmaceutically acceptable salt thereof, or a hydrate or solvate thereof in the medicament includes 120 mg which is calculated as the present compound (free form).

In another most preferred embodiment, single oral dose of the present compound or a pharmaceutically acceptable salt thereof, or a hydrate or solvate thereof in the medicament includes 160 mg which is calculated as the present compound (free form).

In another most preferred embodiment, single oral dose of the present compound or a pharmaceutically acceptable salt thereof, or a hydrate or solvate thereof in the medicament includes 180 mg which is calculated as the present compound (free form).

In another most preferred embodiment, single oral dose of the present compound or a pharmaceutically acceptable salt thereof, or a hydrate or solvate thereof in the medicament includes 200 mg which is calculated as the present compound (free form).

In another most preferred embodiment, single oral dose of the present compound or a pharmaceutically acceptable salt thereof, or a hydrate or solvate thereof in the medicament includes 240 mg which is calculated as the present compound (free form).

In another most preferred embodiment, single oral dose of the present compound or a pharmaceutically acceptable salt thereof, or a hydrate or solvate thereof in the medicament includes 360 mg which is calculated as the present compound (free form).

The medicament comprising the present compound or a pharmaceutically acceptable salt thereof, or a hydrate or solvate thereof, is preferably administered orally once daily. The single oral dose includes preferably, 80 mg, 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, and 400 mg, each of which is calculated as the present compound (free form). And, it also includes the range between these two arbitrarily-selected doses.

The medicament comprising the present compound or a pharmaceutically acceptable salt thereof, or a hydrate or solvate thereof, is preferably administered orally once daily. The single oral dose includes more preferably, 100 mg, 120 mg, 140 mg, 160 mg, 180 mg, 200 mg, 220 mg, 240 mg, 260 mg, 280 mg, and 300 mg, each of which is calculated as the present compound (free form). And, it also includes the range between these two arbitrarily-selected doses.

The medicament comprising the present compound or a pharmaceutically acceptable salt thereof, or a hydrate or solvate thereof, is preferably administered orally once daily. The single oral dose includes even more preferably, 120 mg, 140 mg, 160 mg, 180 mg, 200 mg, 220 mg, 240 mg, 260 mg, and 280 mg, each of which is calculated as the present compound (free form). And, it also includes the range between these two arbitrarily-selected doses.

The medicament comprising the present compound or a pharmaceutically acceptable salt thereof, or a hydrate or solvate thereof, is preferably administered orally once daily. The single oral dose includes the most preferably, 140 mg, 160 mg, 180 mg, 200 mg, 220 mg, 240 mg, and 260 mg, each of which is calculated as the present compound (free form). And, it also includes the range between these two arbitrarily-selected doses.

The medicament comprising the present compound or a pharmaceutically acceptable salt thereof, or a hydrate or solvate thereof, is preferably administered orally once daily. In another preferred embodiment, the single oral dose includes 160 mg, 180 mg, 200 mg, 220 mg, and 240 mg, each of which is calculated as the present compound (free form). And, it also includes the range between these two arbitrarily-selected doses.

The medicament comprising the present compound or a pharmaceutically acceptable salt thereof, or a hydrate or solvate thereof, is preferably administered orally once daily. In another more preferred embodiment, the single oral dose includes 20 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 40 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 60 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 80 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 120 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 160 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 180 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 200 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 240 mg which is calculated as the present compound (free form).

The medicament comprising the present compound or a pharmaceutically acceptable salt thereof, or a hydrate or solvate thereof, is preferably administered orally once daily. In another more preferred embodiment, the single oral dose includes 360 mg which is calculated as the present compound (free form).

The present compound or a pharmaceutically acceptable salt thereof, or a hydrate or solvate thereof, may be administered either orally or parenterally, either directly or in an appropriate dosage form. In a more preferred embodiment, the present compound may be administered orally, either directly or in an appropriate dosage form.

The dosage form includes, for example, a tablet, a capsule, a powder, a granule, a liquid, a suspension, an injection, a patch, a poultice, and the like, but it is not limited to them. The drug formulation is prepared by a common method using pharmaceutically acceptable additives.

As the additive, an excipient, a disintegrant, a binder, a fluidizer, a lubricant, a coating agent, a solubilizer, a solubilizing adjuvant, a thickener, a dispersant, a stabilizing agent, a sweetening agent, a flavor, and the like may be used, depending on purpose. The additive used herein includes, for example, lactose, mannitol, crystalline cellulose, low-substituted hydroxypropylcellulose, corn starch, partially-pregelatinized starch, carmellose calcium, croscarmellose sodium, hydroxypropylcellulose, hydroxypropylmethylcellulose, polyvinyl alcohol, magnesium stearate, sodium stearyl fumarate, polyethylene glycol, propylene glycol, titanium oxide, talc, and the like.

### EXAMPLES

The metabolites used in the following examples were prepared in the following reference examples.

In the present specification, the abbreviations shown below may be used.
Hex: hexane
IPA: isopropyl alcohol
THF: tetrahydrofuran
TFA: trifluoroacetic acid
NaBH(OAc)₃: sodium triacetoxyborohydride
DMF: N,N-dimethylformamide
MeCN: acetonitrile
WSCI-HCl: 1-(3-dimethylaminopropyl)-3-ethylcarbodiimide hydrochloride
HOBt H₂O: 1-hydroxybenzotriazole monohydrate
Me: methyl
Et: ethyl
Boc: tert-butoxycarbonyl

NMR data used for identification of compounds were obtained with JEOL JNM-AL series AL400.

The symbols used in NMR are defined as follows, s: singlet, d: doublet, dd: doublet of doublet, t: triplet, td: triplet of doublet, q: quartet, m: multiplet, br: broad, brs: broad singlet, brm: broad multiplet, and J: coupling constant.

Analytical conditions of LC/MS used for identification of compounds are shown below. Observed mass spectrometry values [MS(m/z)] are shown in [M+H]⁺ or [M+2H]²⁺, and retention times are shown as Rt (min).

### LC/MS measurement method:

Detection apparatus: ACQUITY^{TM} SQ detector (Waters)
HPLC: ACQUITY UPLC^{TM} system
Column: Waters ACQUITY UPLC^{TM} BEH C18 (1.7 µm, 2.1 mm x 30 mm)
Solvent: A: 0.06% formic acid/H₂O, B: 0.06 % formic acid/MeCN Gradient condition: 0.0-1.3 min linear gradient from B 2% to 96%
Flow rate: 0.8 mL/min
UV: 220 nm and 254 nm

Among the compounds of the Reference examples described herein and their synthetic intermediates, analysis of optically active compounds was carried out under the following conditions.

HPLC evaluation system for optical resolution:
Pump: LC-20AD (Shimadzu)
Ditector: SPD-20A (Shimadzu)
Pump: SIL-20A (Shimadzu)
Condition A
Column: CHIRALPAK IE
Size: 0.46 cm I.D. x 25 cm L
Mobile phase: n-Hex/EtOH/i-PrNH₂=90/10/0.1<v/v>
Flow rate: 1.0 mL/min
Temprature: 40°C
Wavelength: 287 nm

### Condition B

Column: CHIRALPAK IE
Size: 0.46 cm I.D. x 25 cm L
Mobile phase: n-Hex/IPA/i-PrNH₂=80/20/0.1<v/v>
Flow rate: 1.0 mL/min
Temprature: 40°C
Wavelength: 285 nm

### Condition C

Column: CHIRALPAK IE
Size: 0.46 cm I.D. x 25 cm L
Mobile phase: MeOH/i-PrNH₂=100/0.1<v/v>
Flow rate: 1.0 mL/min
Temprature: 40°C
Wavelength: 280 nm

### Condition D

Column: CHIRALPAK AD-H
Size: 0.46 cm I.D. x 25 cm L
Mobile phase: n-Hex/IPA/EtOH/i-PrNH₂=85/5/10/0.1<v/v>
Flow rate: 1.0 mL/min
Temprature: 40°C
Wavelength: 280 nm

### Reference example 1

### tert-Butyl 4-(hydroxymethyl)-4-(3-methylbut-2-en-1-yl)piperidine-1-carboxylate

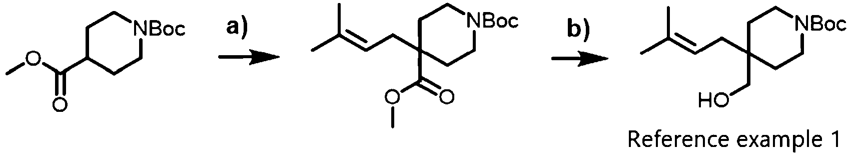

### a) Preparation of 1-tert-butyl 4-methyl 4-(3-methylbut-2-en-1-yl)piperidine-1,4-dicarboxylate

To a solution of 1-tert-butyl 4-methyl piperidine-1,4-dicarboxylate (69.0 g) in tetrahydrofuran (700 mL) was added dropwise 1 mol/L sodium bis(trimethylsilyl)amide (326 mL) at -78°, and the mixture was stirred at the same temperature for one hour. The mixture was warmed to -20°C, and stirred for one hour. Then, 1-bromo-3-methyl-2-butene (42.6 mL) was added dropwise to the mixture at -78°C. The mixture was warmed to -20°C, and stirred for 3 hours. The stirring was continued overnight at room temperature. After the reaction was completed, an aqueous ammonium chloride solution was added to the reaction solution, followed by the addition of water and ethyl acetate. The mixture was extracted with ethyl acetate and water, and the resulting organic layer was washed with brine and dried over sodium sulfate. The solvent was evaporated under reduced pressure, and the resulting residue was purified by silica gel column chromatography (hexane/ethyl acetate) to obtain the title compound (69.0 g).
¹H-NMR (400 MHz, CDCl₃) : 5.01 (1H, t, J = 7.5 Hz), 3.87-3.72 (2H, m), 3.68 (3H, s), 2.86 (2H, dd, J = 2.7, 11.4 Hz), 2.21 (2H, d, J = 7.3 Hz), 2.06 (2H, d, J = 14.2 Hz), 1.69 (3H, s), 1.58 (3H, s), 1.44 (9H, s), 1.43-1.34 (2H, m).
LC-MS; [M+H]⁺ 312.3/Rt(min) 1.24

### b) Preparation of tert-butyl 4-(hydroxymethyl)-4-(3-methylbut-2-en-1-yl)piperidine-1-carboxylate (Reference example 1)

To a solution of lithium aluminum hydride (9.25 g) in tetrahydrofuran (700 mL) was added dropwise a solution of the obtained 1-tert-butyl 4-methyl 4-(3-methylbut-2-en-1-yl)piperidine-1,4-dicarboxylate (69.0 g) in tetrahydrofuran (300 mL) at -78°C. The mixture was stirred at the same temperature for one hour, and then the mixture was warmed to -20°C, and stirred for 5 hours under a nitorogen atomosphere. After the reaction was completed, a saturated aqueous solution of sodium sulfate was added to the reaction solution at 0°C. After stirring at room temperature for one hour, the reaction mixture was dried over magnesium sulfate. The reaction mixture was filtered through Celite, the solvent was evaporated under reduced pressure, and the resulting residue was purified by silica gel column chromatography (hexane/ethyl acetate) to obtain Reference example 1 (58.0 g).
¹H-NMR (400 MHz, CDCl₃) : 5.17 (1H, t, J = 7.8 Hz), 3.48-3.41 (2H, m), 3.46 (2H, s), 3.38-3.31 (2H, m), 2.09 (2H, d, J = 7.8 Hz), 1.73 (3H, s), 1.65 (3H, s), 1.51-1.37 (4H, m), 1.46 (9H, s).
LC-MS; [M+H]⁺ 284.3/Rt(min) 1.08

### Reference example 2

### Racemic mixture of (4'aR,10'bR)-9'-chloro-5',5'-dimethyl-4'a,10'b-dihydro-2'H,4'H,5'H-spiro[piperidine-4,3'-pyrano[3,2-c][1]benzopyran] and (4'aS,10'bS)-9'-chloro-5',5'-dimethyl-4'a,10'b-dihydro-2'H,4'H,5'H-spiro[piperidine-4,3'-pyrano[3,2-c][1]benzopyran]

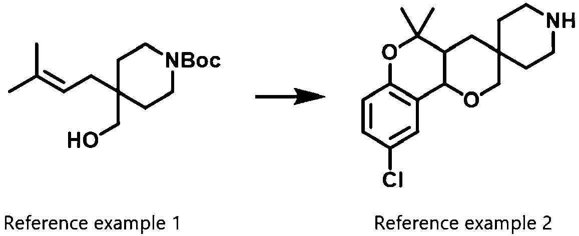

To a solution of Reference example 1 (69.0 g) in dichloromethane (1000 mL) were added 5-chloro-2-hydroxybenzaldehyde (48.0 g), trimethyl orthoformate (100 mL), and para-toluenesulfonic acid hydrate (1.90 g) in that order at 0°C, and the mixture was stirred at 0°C under a nitrogen atmosphere for 5 hours. The solvent was distilled off under reduced pressure, and to the obtained residue was added a 4 mol/L hydrochloric acid/ethyl acetate solution (300 mL) at 0°C, followed by stirring at 0°C for 1 hour. The solvent was distilled off under reduced pressure, and the resulting residue was purified by amine silica gel column chromatography (chloroform/methanol) to give the racemic mixture of Reference example 2 (61.5 g).
¹H-NMR (400 MHz, CDCl₃) : 7.38 (1H, s), 7.11 (1H, d, J = 8.6 Hz), 6.70 (1H, d, J = 8.6 Hz), 4.12 (1H, d, J = 11.4 Hz), 4.08 (1H, d, J = 10.9 Hz), 3.31 (1H, d, J = 10.9 Hz), 2.90-2.86 (3H, m), 2.81-2.77 (1H, m), 1.91-1.80 (3H, m), 1.57-1.53 (1H, m), 1.39-1.37 (2H, m), 1.38 (3H, s), 1.17 (3H, s), 1.11-1.05 (1H, m).
LC-MS; [M+H]⁺ 322.2/Rt(min) 0.77

The obtained Reference example 2 was subjected to optical resolution to obtain Reference examples 3 and 4, which are optically active forms of Reference example 2.

### Reference example 3:

Condition A; Rt (min) = 10.35
LC-MS; [M+H]⁺ 322.2/Rt (min) 0.77

### Reference example 4:

Condition A; Rt (min) = 12.07
LC-MS; [M+H]⁺ 322.2/Rt (min) 0.77

### Reference examples 5 - 15

Using the corresponding starting compounds, the compounds of Reference examples 5 to 15 shown in the table below were obtained in the same manner as in Reference example 2.

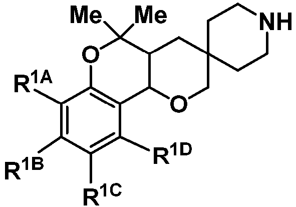

**[Table 1]**

| Ref. ex. | R^{1A} | R^{1B} | R^{1C} | R^{1D} | racemic or chiral | Rt (min) (condition) | LC-MS: [M+H]⁺/Rt (min) |
|---|---|---|---|---|---|---|---|
| 5 | Cl | H | H | H | racemic | - | 322.5/0.72 |
| 6 | Br | H | H | H | racemic | - | 366.2/0.74 |
| 7 | H | Cl | H | H | racemic | - | 322.5/0.75 |
| 8 | H | Cl | H | H | chiral | 8.09 (Condition C) | 322.5/0.74 |
| 9 | H | Cl | H | H | chiral | 11.27 (Condition C) | 322.5/0.76 |
| 10 | H | Br | H | H | racemic | - | 366.2/0.77 |
| 11 | H | H | F | H | racemic | - | 306.5/0.65 |
| 12 | H | H | F | H | chiral | 6.07 (Condition B) | 306.5/0.63 |
| 13 | H | H | F | H | chiral | 7.35 (Condition B) | 306.5/0.65 |
| 14 | H | H | Br | H | racemic | - | 366.2/0.75 |
| 15 | H | H | N₃ | H | racemic | - | 329.3/0.78 |

The NMR data for the compound of Reference example 7 in the above table are shown below.

### Reference example 7

¹H-NMR (400 MHz, CDCl₃): 7.33 (1H, d, J = 9.2 Hz), 6.88 (1H, d, J = 8.4 Hz), 6.80 (1H, s), 4.14-4.07 (2H, m), 3.32 (1H, d, J = 11.6 Hz), 2.90-2.80 (4H, m), 1.93-1.82 (2H, m), 1.58-1.51 (1H, m), 1.38-1.34 (1H, m), 1.36 (3H, s), 1.27-1.19 (2H, m), 1.24 (3H, s), 1.12-1.06 (1H, m).

Preparation of tert-butyl {2-[(4'aR,10'bR)-8'-chloro-5',5'-dimethyl-4'a,10'b-dihydro-2'H,4'H,5'H-spiro[piperidine-4,3'-pyrano[3,2-c][1]benzopyran]-1-yl]ethyl}carbamate

### (Reference example 16)

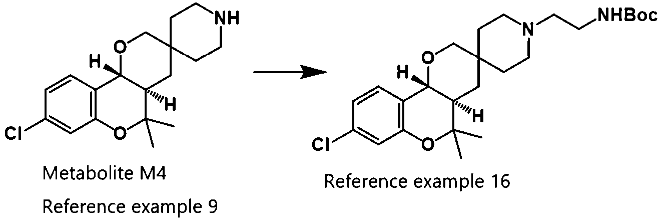

To a solution of Reference example 9 (6.42 g) which is metabolite M4 in acetonitrile (100 mL) were added triethylamine (2.8 mL), potassium carbonate (2.76 g) and tert-butyl (2-bromoethyl)carbamate (4.92 g), and the mixture was stirred at 85°C for 5 hours. After filtration, the solvent was evaporated under reduced pressure and the resulting residue was purified by silica gel column chromatography (chloroform/methanol=1/0 to 25/1) to give Reference example 16 (9.10 g).
¹H-NMR (400 MHz, CDCl₃): 7.32 (1H, dd, J = 8.8, 0.8 Hz), 6.86 (1H, dd, J = 8.4, 2.0 Hz), 6.78 (1H, d, J = 2.0 Hz), 4.95 (1H, bs), 4.08-4.02 (2H, m), 3.30 (1H, d, J = 11.2 Hz), 3.22-3.20 (2H, m), 2.46-2.38 (5H, m), 2.31-2.27 (1H, m), 1.89-1.82 (3H, m), 1.62 (2H, m), 1.45 (9H, s), 1.43-1.39 (1H, m), 1.36 (3H, s), 1.17 (3H, s), 1.11-1.03 (1H, m). (NMR model: Bruker-400)

### Reference example 18

### Preparation of N-2-[(4'aR,10'bR)-8'-chloro-5',5'-dimethyl-4'a,10'b-dihydro-2'H,4'H,5'H-spiro[piperidine-4,3'-pyrano[3,2-c][1]benzopyran]-1-yl]ethyl-N²-formylglycinamide (metabolite M2)

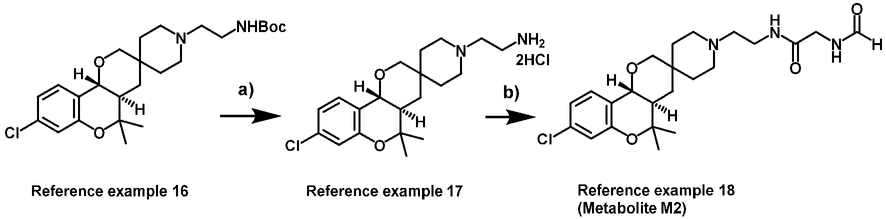

a) To a solution of Reference example 16 (9.10 g) in chloroform (100 mL) was added 4M hydrogen chloride/cyclopentyl methyl ether solution (48.4 mL), and the mixture was stirred at room temperature for 2 hours. The solvent was evaporated under reduced pressure, chloroform (100 mL) was added to the residue, and the solvent was evaporated under reduced pressure again (repeated twice in total) to obtain Reference Example 17 (9.76 g), which was used as it was in the next reaction.
b) To a solution of Reference example 17 (9.76 g, assumed to be 19.6 mmol) in dry chloroform (100 mL) were added N-formylglycine (2.42 g), 1-hydroxybenzotriazole (3.17 g), N-methylmorpholine (10.3 mL), and 1-ethyl-3-(3-dimethylaminopropyl)carbodiimide hydrochloride (5.62 g) at 0°C, and the mixture was warmed to room temperature, and stirred for 15 hours. The mixture was diluted with chloroform, and the organic layer was washed with saturated aqueous sodium bicarbonate and brine, and then dried over anhydrous sodium sulfate. The solvent was evaporated under reduced pressure, and the resulting residue was purified by silica gel column chromatography (chloroform/methanol=1/0 to 6.25/1) and further purified by amino silica gel column chromatography (chloroform/methanol=1/0 to 50/1) to give Reference example 18 (7.32 g).
¹H-NMR (400 MHz, DMSO-d6) : 8.24-8.18 (1H, m), 8.05 (1H, s), 7.82-7.76 (1H, m), 7.29 (1H, d, J = 7.8 Hz), 6.88 (1H, dd, J = 8.0, 2.0 Hz), 6.78 (1H, d, J = 2.0 Hz), 4.12 (1H, d, J = 11.0 Hz), 3.89 (1H, d, J = 11.0 Hz), 3.68 (2H, d, J = 6.0 Hz),3.31-3.28 (1H, m), 3.15 (2H, q, J = 6.4 Hz), 2.40-2.24 (6H, m), 1.76-1.48 (4H, m), 1.36-1.30 (2H, m), 1.32 (3H, s), 1.13 (3H, s), 1.16-1.06 (1H, m).
(JEOL JNM-AL series AL400)

### Reference example 19

### Preparation of 2-[(4'aR,10'bR)-8'-chloro-5',5'-dimethyl-4'a,10'b-dihydro-2'H,4'H,5'H-spiro[piperidine-4,3'-pyrano[3,2-c][1]benzopyran]-1-yl]ethan-1-ol (metabolite M)

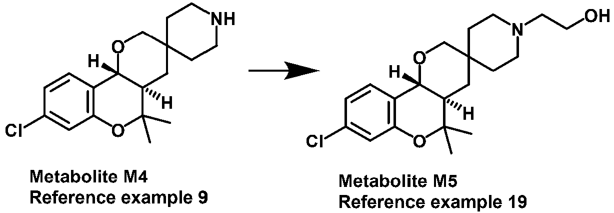

To a solution of metabolite M4 (3.1 g) in acetonitrile (60 mL) were added potassium carbonate (3.99 g) and 2-bromoethanol (0.821 mL), and the mixture was stirred at 50°C for 2 hours. After filtration, the solvent was evaporated under reduced pressure and the resulting residue was purified by amino silica gel column chromatography (ethyl acetate) to obtain Reference example 19 (3.05 g).
¹H-NMR (400 MHz, DMSO-d6) : 7.29 (1H, d, J = 8.0 Hz), 6.88 (1H, dd, J = 8.4, 2.4 Hz), 6.78 (1H, d, J = 2.4 Hz), 4.31 (1H, t, J = 5.6 Hz), 4.11 (1H, d, J = 10.4 Hz), 3.88 (1H, d, J = 10.8 Hz), 3.45 (2H, q, J = 6.4 Hz), 3.31-3.27 (1H, m), 2.41-2.24 (6H, m), 1.76-1.47 (4H, m), 1.36-1.29 (2H, m), 1.32 (3H, s), 1.13 (3H, s), 1.13-1.06 (1H, m).

### (JEOL JNM-AL series AL400)

The present invention is described in more detail below with examples, but, the present invention is not limited thereto. The doses (mg) described in the following sections, "1. Clinical trial in patients with recurrent malignant glioma classified as WHO grade III or IV", "2. Clinical trial in patients with recurrent malignant glioma classified as WHO grade II, III or IV", and Examples 1 to 15 are all doses (mg) converted to the amount of the present compound (free form). For example, the statement "supplied as a capsule containing 20 mg of Compound 1" means "supplied as a capsule containing 20 mg of the active ingredient calculated in terms of the present compound", and the capsule contains 29.4 mg of Compound 1. For example, the expression "initial starting dose of 20 mg/day" means "initial starting dose of 20 mg/day calculated in terms of the present compound," and indicates that Compound 1 was administered at an initial starting dose of 29.4 mg/day. For example, the expression "20 mg QD" means "20 mg QD calculated in terms of the present compound," and indicates that Compound 1 is administered at 29.4 mg QD.

### 1. Clinical Trial in Patients with Recurrent Malignant Glioma Classified as WHO grade III or IV

### (1) Clinical trial outline

The present study is a Phase 1 dose-escalation trial with a dose-expansion part designed to evaluate the safety, pharmacokinetics (PK), pharmacodynamics (PD), and preliminary antitumor activity of Compound 1 following oral administration in patients with recurrent malignant glioma classified as WHO grade III or IV. In the present study, Compound 1 was orally administered daily. Each treatment cycle was defined as 28 days, during which safety and treatment response were evaluated. Administration to each subject was continued until unacceptable toxicity occurred, consent was withdrawn, the subject was lost to follow-up, or discontinuation was determined by the investigator and/or the sponsor.

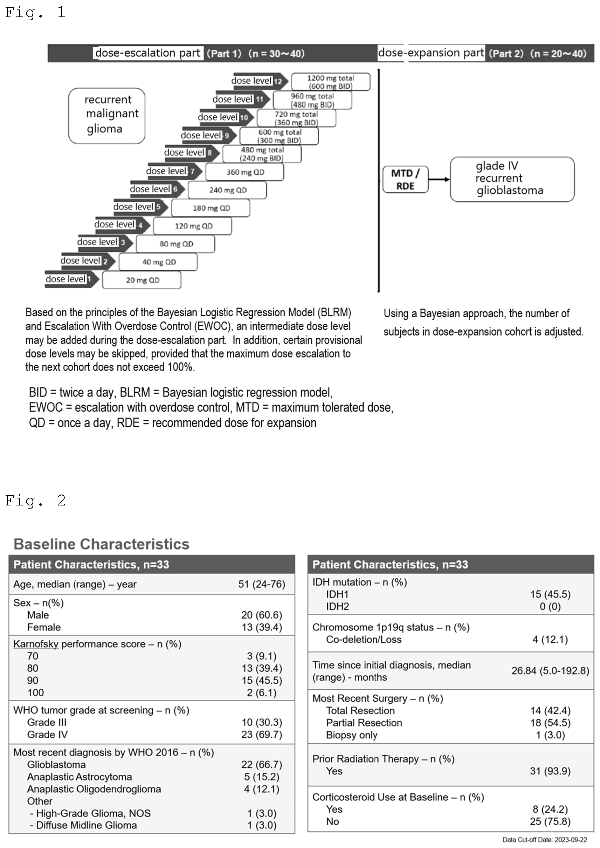

An overview of the overall study design is shown in Figure 1. The present study consists of two parts: a dose-escalation part (Part 1) and a dose-expansion part (Part 2). In the dose-escalation part, the dose of Compound 1 was escalated in patients with malignant glioma classified as WHO grade III or IV to determine the maximum tolerated dose (MTD) and/or the recommended dose for expansion (RDE), which may be lower than the MTD. Compound 1 was supplied as a capsule containing Compound 1 equivalent 20 mg or 60 mg of the present compound.

Once the MTD and/or RDE is established, the dose-expansion part of the study is initiated. In the dose-expansion part, approximately 20-40 patients with recurrent glioblastoma (GBM) classified as WHO grade IV are enrolled to evaluate the preliminary clinical activity, as well as the safety and tolerability, of Compound 1. Using a Bayesian approach, (a) the posterior probability that the objective response rate (ORR) is ≥15% is evaluated after at least 10 ORR-evaluable subjects have been enrolled, and (b) the result is used as guidance for subject enrollment into the dose-expansion part. At the end of the dose-expansion part, the dose selected by the sponsor in consultation with the investigator(s), based on the safety, clinical activity, pharmacokinetic (PK), and pharmacodynamic (PD) data collected in the present study, is determined as the recommended Phase 2 dose (RP2D).

Approximately 30-40 subjects were enrolled in the dose-escalation part of the present study. In addition, approximately 20-40 subjects are enrolled in the dose-expansion part.

### (2) Overall study design

The initial starting dose of 20 mg/day (expressed as the present compound) was determined in accordance with the ICH S9 guideline of International Council for Harmonisation of Technical Requirements for Pharmaceuticals for Human Use (ICH). Based on a 4-week mouse toxicity study conducted in compliance with Good Laboratory Practice (GLP), the dose causing severe toxicity in 10% of the test animals (STD10) was determined to be 50 mg/kg. Applying a safety factor of 10 and interspecies scaling, the starting dose for this first-in-human (FIH) study was calculated to be 0.4 mg/kg, corresponding to a fixed dose of 24 mg for a 60-kg human. In a 4-week non-human primate toxicity study conducted in compliance with Good Laboratory Practice (GLP), the highest non-severely toxic dose (HNSTD) was determined to be 7.5 mg/kg. Applying a safety factor of 6 and interspecies scaling, the starting dose in humans was estimated to be 0.4 mg/kg, corresponding to a fixed dose of 24 mg. The final recommended starting dose for the first-in-human (FIH) study was rounded to 20 mg/day. This recommended starting dose exceeds the minimum pharmacologically active dose in humans of 15 mg, which corresponds to the human equivalent dose (HED) of 3 mg/kg at which a consistent tumor shrinkage effect was observed in mice bearing U-87 GBM xenografts.

The provisional dose levels are shown in Table 2.

**[Table 2]**

| Dose level | Dose calculated in terms of the present compound |
|---|---|
| 1 | 20 mg QD |
| 2 | 40 mg QD |
| 3 | 80 mg QD |
| 4 | 120 mg QD |
| 5 | 180 mg QD |
| 6 | 240 mg QD |
| 7 | 360 mg QD |
| 8 | A total of 480 mg (240 mg BID) |
| 9 | A total of 600 mg (300 mg BID) |
| 10 | A total of 720 mg (360 mg BID) |
| 11 | A total of 960 mg (480 mg BID) |
| 12 | A total of 1200 mg (600 mg BID) |
| Note: Based on the principles of the Bayesian Logistic Regression Model (BLRM) and Escalation With Overdose Control (EWOC), an intermediate dose level may be added during the dose-escalation part. In addition, certain provisional dose levels may be skipped, provided that the maximum dose escalation to the next cohort does not exceed 100%. | |

An alternative dosing schedule, such as twice-daily (BID) administration, was initiated at a total daily dose of 480 mg in order to reduce pill burden (*see*, the table of provisional dose levels for details). A change in the dosing schedule from once daily (QD) to twice daily (BID) is not expected to affect the steady-state mean exposure at the same total daily dose.

### (3) Dose-escalation part

The maximum tolerated dose (MTD) was estimated based on the occurrence of dose-limiting toxicities (DLTs) during the DLT evaluation period (Cycle 1; 28 days from the start of investigational drug administration), using an adaptive Bayesian logistic regression model (BLRM) with escalation with overdose control (EWOC) as the guideline for dose escalation. Each dose level was evaluated in cohorts of 1-6 patients. In all cohorts, administration of the investigational drug to the second and subsequent patients was permitted after completion of all safety assessments on Day 8 of Cycle 1 in the first patient. If a DLT was observed at a given dose level, at least three patients were evaluated at that dose level. At dose level 2 in the dose-escalation part, at least three patients were required to be evaluated before escalation to a higher dose level. After completion of each cohort, the probability of observing a DLT at each dose level in the present study was updated using the BLRM based on cumulative data from the preceding cohorts, and the MTD was estimated.

The safety at each dose level was evaluated at a Dose Determination Meeting (DDM) composed of the sponsor's representatives (the medical monitor serving as chair of the meeting, the clinical development lead, the lead statistician, and representatives from pharmacovigilance, clinical pharmacology, and biomarker functions), as well as the principal investigators participating in the present study. The primary objective of the DDM was to determine the dose and dosing schedule, as well as the number of additional patients to be treated with the investigational drug in the present study. At the conclusion of the DDM, the medical monitor, the representative from pharmacovigilance function, and the principal investigators reached agreement on the subsequent dosing and enrollment decisions in accordance with the study protocol.

The decision to escalate, de-escalate, or maintain the dose in the next cohort was made based on the recommendation from the BLRM and a review of other available data, including PK and PD data from all patients who received the investigational drug; patient characteristics, medical history, and prior anticancer therapies; investigational drug administration data such as dose reductions or discontinuations; clinical laboratory results; imaging findings and performance status assessments; adverse events (AEs) and serious adverse events (SAEs); and other clinical data, including whether DLTs had occurred.

Intra-patient dose escalation was optional and could be implemented at the discretion of the principal investigator, etc. if a higher dose had been shown to be safe and the patient was considered likely to benefit from dose escalation. Intra-patient dose escalation was not permitted at any time within the first two cycles at the initially assigned dose level. Before a patient could receive Compound 1 at a higher dose, tolerability at the lower dose has to be confirmed during at least two cycles of treatment (*i.e*., no Compound 1-related non-hematologic or hematologic toxicities of CTCAE Grade ≥2 occurred at the initially assigned lower dose). Furthermore, the new higher dose to be administered to the patient has to be a dose level that has already been evaluated and is not above the MTD. There is no limit on the number of times the dose of Compound 1 could be escalated for an individual patient.

The following rules were applied to any further dose escalations after the first intra-patient dose escalation. That is, the patient must not have experienced any Compound 1-related non-hematologic or hematologic toxicities of CTCAE Grade ≥2 at the lower dose during at least the two most recent treatment cycles. In addition, the higher dose under consideration has to have been adequately evaluated and demonstrated not to exceed the MTD.

### (4) Dose-expansion part

Once the MTD and/or RDE is established, the dose-expansion part of the study is initiated. In the dose-expansion part, approximately 20-40 patients with recurrent GBM classified as WHO grade IV are enrolled to evaluate the preliminary antitumor activity of Compound 1 administered at the MTD and/or RDE and to further assess its safety and tolerability. At the end of the dose-expansion part, the dose selected by the sponsor in consultation with the investigator, etc., based on the safety, clinical activity, PK, and PD data collected in the present study, is determined as RP2D. Using a Bayesian approach, (a) the posterior probability that the objective response rate (ORR) is ≥15% is evaluated after at least 10 ORR-evaluable subjects have been enrolled, and (b) the number of subjects in the dose-expansion part may be adjusted accordingly.

### (5) Description of dose-escalation and dose-expansion parts

Compound 1 was supplied as 20 mg and 60 mg capsules. Compound 1 was administered orally daily preferably with approximately 200 mL of water or about half a glass of water. Compound 1 was administered after a fasting period of at least 6 hours, and patients were required to remain fasting for 1 hour after dosing. For BID dosing, the morning dose of Compound 1 was administered after a fasting period of at least 6 hours, and patients were required to remain fasting for 1 hour after dosing. For the evening dose, Compound 1 was administered after a fasting period of 2 hours, and patients were required to remain fasting for 1 hour after dosing. The evening dose was administered approximately 12 hours after the morning dose of Compound 1.

In the dose-escalation part, patients continued treatment at the assigned dose until the discontinuation criteria were met.

Patient compliance with dosing was monitored at the time points specified by the sponsor based on patient dosing diaries and/or investigational drug accountability records. Patients recorded the administration of Compound 1 using a patient dosing diary.

In the dose-expansion part, patients continue treatment at the MTD and/or RDE determined in the dose-escalation part until the discontinuation criteria are met. At the end of the dose-expansion part, the dose selected by the sponsor in consultation with the principal investigators, based on the safety, clinical activity, PK, and PD data collected during this study, is determined as the RP2D.

### Example 1: Clinical Safety Assessment of Compound 1 (1)

As of the data cutoff date of April 21, 2023, Compound 1 had been administered to 23 patients. The dose levels were as follows: 20 mg (n=2), 40 mg (n=4), 80 mg (n=3), 120 mg (n=6), 180 mg (n=3), and 240 mg (n=5). Among the 23 patients, 21 (91.3%) had discontinued treatment with Compound 1 as of the data cutoff date. The most common reason for discontinuation of Compound 1 was disease progression (17 patients, 73.9%). In addition, 9 patients discontinued the study. The reasons for study discontinuation were death in 4 patients (all due to disease progression), completion of the 12-month survival follow-up in 2 patients, and study termination at the investigational site due to the departure of the principal investigator in 3 patients. Treatment-emergent adverse events (TEAEs) were observed in all 23 patients who received Compound 1, and most of the events were reported in a single patient each. TEAEs reported in more than 10% of patients are summarized in Table 3. In Table 3, the dose levels represent doses equivalent to the present compound. For example, "20 mg of Compound 1" means 20 mg expressed as the equivalent dose of the present compound, which corresponds to an actual administered dose of 29.4 mg of Compound 1. Among the 23 patients, 16 (69.6%) experienced TEAEs considered to be related to the study drug. Among these, TEAEs reported in two or more patients were nausea, increased alanine aminotransferase, increased aspartate aminotransferase, dry skin, decreased platelet count, vomiting, constipation, and decreased appetite. Grade ≥3 TEAEs were reported in 13 patients (56.5%). The most frequently reported events were seizure (n=4, 17.4%), decreased neutrophil count (n=3, 13.0%), aphasia, headache, decreased lymphocyte count, and decreased white blood cell count (each n=2, 8.7%). A Grade ≥3 TEAE considered related to Compound 1 was reported in one patient (dry skin). Serious TEAEs were reported in 6 patients (26.1%), including seizure in three patients (in the 20 mg, 120 mg, and 180 mg groups), aphasia in one patient (120 mg group), disease progression in one patient (40 mg group), and neoplasm progression in one patient (240 mg group). None of these serious TEAEs were considered to be related to Compound 1. In addition, two patients died during the study due to TEAEs (one due to disease progression in the 40 mg group and one due to neoplasm progression in the 240 mg group). Both events were assessed as not related to Compound 1. No dose-limiting toxicities were observed at any dose level.

**[Table 3]**

| Preferred Term | Comp. 1 20 mg (N=2) n (%) | Comp. 1 40 mg (N=4) n (%) | Comp. 1 80 mg (N=3) n (%) | Comp. 1 120 mg (N=6) n (%) | Comp. 1 180 mg (N=3) n (%) | Comp. 1 240 mg (N=5) n (%) | Total (N=23) n (%) |
|---|---|---|---|---|---|---|---|
| Number of patients with any TEAE | 2 (100) | 4 (100) | 3 (100) | 6 (100) | 3 (100) | 5 (100) | 23 (100) |
| increased alanine aminotransferase | 1 (50.0) | 1 (25.0) | 2 (66.7) | 2 (33.3) | 2 (66.7) | 4 (80.0) | 12 (52.2) |
| nausea | 1 (50.0) | 2 (50.0) | 2 (66.7) | 2 (33.3) | 1 (33.3) | 3 (60.0) | 11 (47.8) |
| constipation | 0 | 1 (25.0) | 0 | 2 (33.3) | 1 (33.3) | 3 (60.0) | 7 (30.4) |
| headache | 0 | 1 (25.0) | 1 (33.3) | 2 (33.3) | 0 | 3 (60.0) | 7 (30.4) |
| increased aspartate aminotransferase | 1 (50.0) | 2 (50.0) | 0 | 1 (16.7) | 0 | 2 (40.0) | 6 (26.1) |
| decreased neutrophil count | 0 | 2 (50.0) | 2 (66.7) | 1 (16.7) | 1 (33.3) | 0 | 6 (26.1) |
| vomiting | 0 | 1 (25.0) | 0 | 2 (33.3) | 1 (33.3) | 2 (40.0) | 6 (26.1) |
| dry skin | 1 (50.0) | 0 | 0 | 1 (16.7) | 0 | 3 (60.0) | 5 (21.7) |
| decreased lymphocyte count | 0 | 2 (50.0) | 0 | 0 | 1 (33.3) | 2 (40.0) | 5 (21.7) |
| seizure | 1 (50.0) | 1 (25.0) | 0 | 1 (16.7) | 1 (33.3) | 1 (20.0) | 5 (21.7) |
| hypertrigly-ceridaemia | 0 | 1 (25.0) | 0 | 1 (16.7) | 1 (33.3) | 1 (20.0) | 4 (17.4) |
| decreased platelet count | 0 | 2 (50.0) | 1 (33.3) | 0 | 1 (33.3) | 0 | 4 (17.4) |
| decreased appetite | 0 | 1 (25.0) | 0 | 0 | 1 (33.3) | 1 (20.0) | 3 (13.0) |
| fall | 0 | 1 (25.0) | 0 | 1 (16.7) | 1 (33.3) | 0 | 3 (13.0) |
| hyponatremia | 0 | 1 (25.0) | 1 (33.3) | 1 (16.7) | 0 | 0 | 3 (13.0) |
| decreased white blood cell count | 0 | 2 (50.0) | 1 (33.3) | 0 | 0 | 0 | 3 (13.0) |
| TEAE: Adverse event that emerged or worsened after administration of the study drug. | | | | | | | |
| Note: Based on MedDRA ver. 24.1 | | | | | | | |
| Source: t_14_3_2_x_ae_by_pt.sas; t_14_3_2_x_ae_by_pt_grd3.sas | | | | | | | |
| Data extraction date: April 21, 2023; Data cutoff date: April 21, 2023. | | | | | | | |

### Example 2: Interim Report of the Phase 1 First-in-Human Study of Compound 1

### (1) Baseline Patients Characteristics

As of September 2023, 33 patients have been enrolled. The median age was 51. Approximately 67% of patients with glioblastoma according to the WHO 2016 criteria, and around 45% of the patients had an IDH1 mutation. Most patients received complete resection and prior radiation therapy. (Figure 2)

### (2) Dose Escalation and Dose Limiting Toxicities

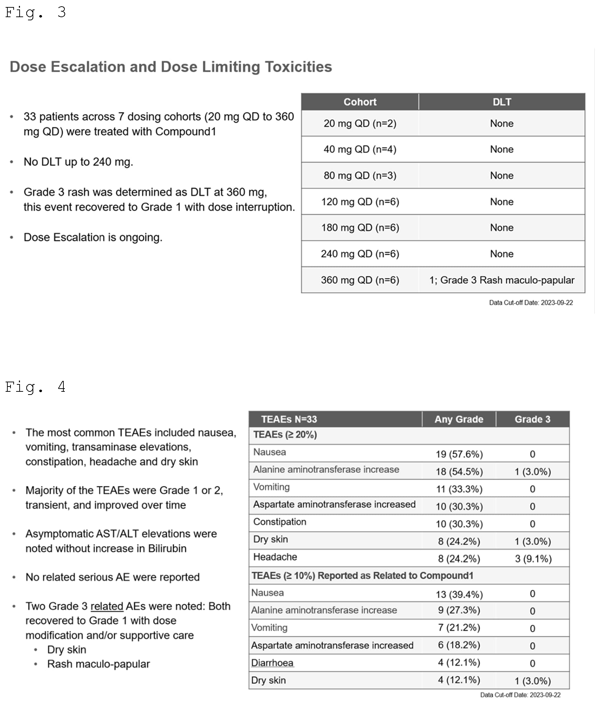

As of September 2023, the dose levels ranging from 20 mg to 480 mg have completed safety evaluation. No dose limiting toxicity reported from 20 mg up to 240 mg. At 360 mg cohort, one patient experienced a Grade 3 maculo-papular rash, which was considered a DLT. This event recovered to grade 1 with dose interruption. (Figure 3)

### (3) Safety

As of September 2023, the most common TEAEs were Grade 1 or 2 nausea, vomiting, and asymptomatic ALT/AST elevations without increase in bilirubin. There were no study drug-related SAEs reported. Only 2 study drug related Grade 3 AEs, dry skin, and rash, were reported, they were manageable with dose modification or supportive care. (FIgure 4)

### (4) Pharmacodynamics - Lathosterol/Zymostenol (L/Z) ratio

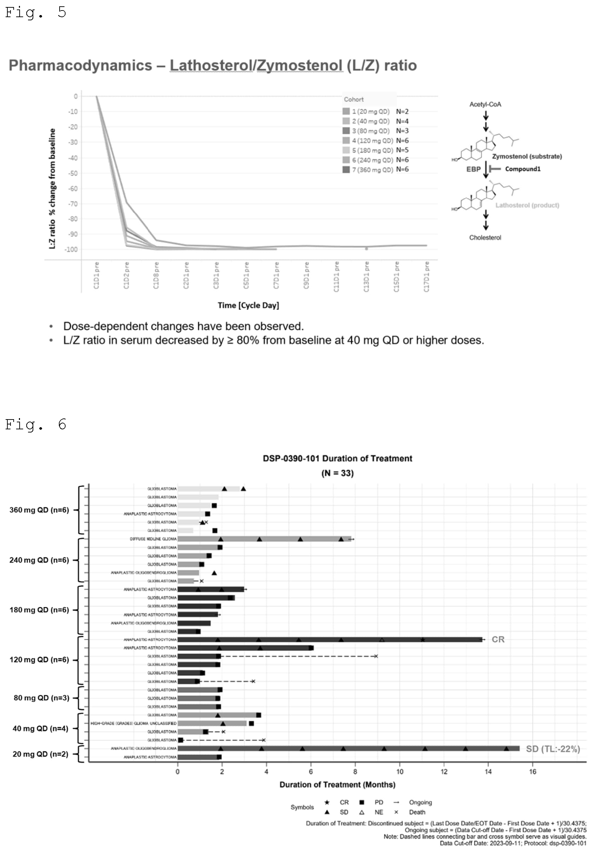

As of September 2023, pharmacodynamic assessments had clearly shown that Compound 1 inhibited the target, at least in serum. Notably, the ratio of lathosterol and zymostenol, intermediates in cholesterol biosynthesis mediated by EBP, decreased by over 80% starting from 40 mg QD cohort. (Figure 5)

### (5) Duration of treatment - Dose escalation part (n=33)

While the patients enrolled in the dose escalation part were heterogeneous, it was worth noting that 2 patients with Grade III glioma with IDH mutation, astrocytoma and oligodendroglioma, had reduction in target lesion and stayed on treatment for over a year. One of these two patients achieved a complete response after 1 year of study treatment (Figure 6).

### (6) Summary of the preliminary aAntitumor activity

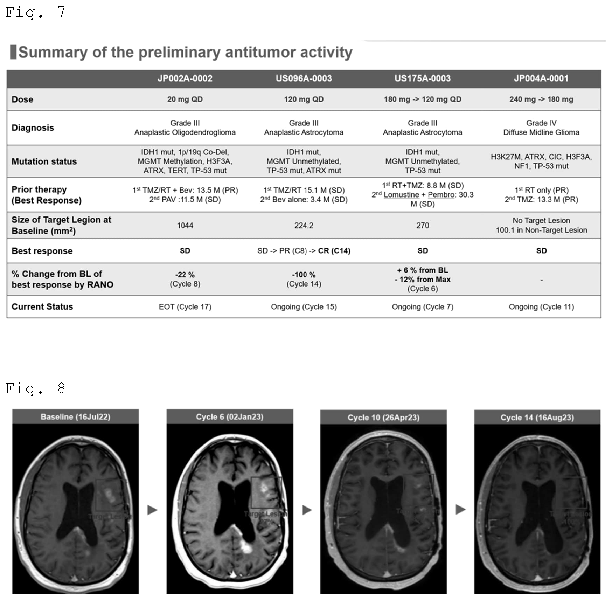

As of September 2023, preliminary antitumor activity of Compound 1 monotherapy has been observed in 4 patients during the Dose Escalation Part. Three of them had Grade III glioma with IDH mutant. And, all patients had received at least two prior lines of therapy (Figure 7).

A patient with WHO grade III astrocytoma with IDH mutation had received 120 mg of Compound 1. This patient achieved complete response (CR) after more than one year of treatment (Figure 8).

### (7) Conclusions

Compound 1 is an investigational small molecule inhibitor of EBP, which is a novel target that regulates cholesterol biosynthesis and modulates the LXR downstream signaling pathway. Compound 1 is given orally and has been well tolerated thus far and has shown a favorable dose dependent PK and PD response. The most common TEAEs were Grade 1 or 2 nausea, vomiting, and asymptomatic ALT/AST elevations (bilirubin was normal range). One DLT (Grade 3 maculopapular rash) at 360 mg QD recovered to Grade 1 with dose interruption. Two patients with WHO Grade III glioma harboring IDH mutations showed tumor reductions of 22% and 100%, respectively, and had remained on study for 15 and 13 months, respectively, as of the data cutoff date. Dose escalation will be continued, and a dose expansion part in patients with IDH-mutant glioma is planned to be initiated.

### Example 3: Clinical Safety Assessment of Compound 1 (2)

As of the data cutoff date of April 21, 2024, Compound 1 had been administered to 39 patients. The dose levels were as follows: 20 mg QD (n=2), 40 mg QD (n=4), 80 mg QD (n=3), 120 mg QD (n=6), 180 mg QD (n=6), 240 mg QD (n=6), 360 mg QD (n=6), and 480 mg daily (240 mg BID) (n=6). Among the 39 patients, 37 (94.9%) had discontinued treatment with Compound 1 as of the data cutoff date. The most common reason for discontinuation of Compound 1 was disease progression (27 patients, 69.2%). Treatment-emergent adverse events (TEAEs) were observed in all 39 patients who received Compound 1. TEAEs reported in more than 10% of patients are summarized in Table 4. In Table 4, the dose levels represent doses equivalent to the present compound. For example, "20 mg of Compound 1" means 20 mg expressed as the equivalent dose of the present compound, which corresponds to an actual administered dose of 29.4 mg of Compound 1. Among the 39 patients, 30 (76.9%) experienced TEAEs considered to be related to the study drug. Among these, TEAEs reported in two or more patients were nausea, increased alanine aminotransferase, vomiting, increased aspartate aminotransferase, constipation, diarrhea, dry skin, maculopapular rash, alopecia, fatigue, decreased platelet count, and decreased appetite. Grade ≥3 TEAEs were reported in 23 patients (59.0%). The most frequently reported events were seizure (n=5, 12.8%), fatigue (n=4, 10.3%), aphasia, headache, decreased lymphocyte count, decreased neutrophil count (each n=3, 7.7%), and dry skin, maculopapular rash, decreased white blood cell count, and increased alanine aminotransferase (each n=2, 5.1%). Grade ≥3 TEAEs considered related to Compound 1 were dry skin (n=2, 5.1%), maculopapular rash (n=2, 5.1%), and fatigue (n=1, 2.6%). Serious TEAEs were reported in 9 patients (23.1%), including seizure in four patients (one in the 20 mg QD group, one in the 120 mg QD group, and two in the 180 mg QD group), disease progression in one patient (40 mg QD group), aphasia in one patient (120 mg QD group), neoplasm progression in one patient (240 mg QD group), and confusional state and fatigue in one patient each (480 mg daily [240 mg BID] group). Among these serious TEAEs, nine events were considered to be related to Compound 1. Two patients died during the study due to TEAEs: one due to disease progression in the 40 mg QD group and one due to neoplasm progression in the 240 mg QD group. None of these serious TEAEs were considered to be related to Compound 1. No dose-limiting toxicities (DLTs) were observed from 20 mg QD to 240 mg QD. A DLT (Grade 3 maculopapular rash) was reported in one patient at 360 mg QD. The event recovered to Grade 1 with treatment interruption. At the 480 mg daily (240 mg BID) dose level, DLTs were reported in two patients: Grade 3 maculopapular rash in one patient and Grade 2 nausea and vomiting in one patient. The Grade 3 maculopapular rash improved to Grade 1 after discontinuation of Compound 1. The Grade 2 nausea and vomiting improved to Grade 1 with dose reduction and treatment interruption. Based on these results, the maximum tolerated dose (MTD) was determined to be 480 mg daily (240 mg BID), and dose escalation was completed.

**[Table 4]**

| Preferred Term | Comp. 1 20 mg QD (N=2) n (%) | Comp. 1 40 mg QD (N=4) n (%) | Comp. 1 80 mg QD (N=3) n (%) | Comp. 1 120 mg QD (N=6) n (%) | Comp. 1 180 mg QD (N=6) n (%) | Comp. 1 240 mg QD (N=6) n (%) | Comp. 1 360 mg QD (N=6) n (%) | Comp. 1 480 mg daily (240 mg BID) (N=6) n (%) | Total (N=39) n (%) |
|---|---|---|---|---|---|---|---|---|---|
| Number of Patients with Any TEAE | 2 (100) | 4 (100) | 3 (100) | 6 (100) | 6 (100) | 6 (100) | 6 (100) | 6 (100) | 39 (100) |
| increased alanine aminotransferase | 1 (50.0) | 1 (25.0) | 2 (66.7) | 2 (33.3) | 4 (66.7) | 6 (100) | 4 (66.7) | 5 (83.3) | 25 (64.1) |
| nausea | 1 (50.0) | 2 (50.0) | 2 (66.7) | 2 (33.3) | 2 (33.3) | 4 (66.7) | 6 (100) | 2 (33.3) | 21 (53.8) |
| vomiting | 0 | 1 (25.0) | 0 | 2 (33.3) | 2 (33.3) | 2 (33.3) | 4 (66.7) | 3 (50.0) | 14 (35.9) |
| increased aspartate aminotransferase | 1 (50.0) | 2 (50.0) | 0 | 1 (16.7) | 2 (33.3) | 2 (33.3) | 3 (50.0) | 2 (33.3) | 13 (33.3) |
| constipation | 0 | 1 (25.0) | 0 | 2 (33.3) | 2 (33.3) | 3 (50.0) | 2 (33.3) | 1 (16.7) | 11 **(28.2)** |
| dry skin | 1 (50.0) | 0 | 0 | 1 (16.7) | 1 (16.7) | 4 (66.7) | 0 | 3 (50.0) | 10 (25.6) |
| fall | 0 | 1 (25.0) | 0 | 1 (16.7) | 2 (33.3) | 0 | 2 (33.3) | 3 (50.0) | 9 (23.1) |
| headache | 0 | 1 (25.0) | 1 (33.3) | 2 (33.3) | 0 | 4 (66.7) | 1 (16.7) | 0 | 9 (23.1) |
| fatigue | 0 | 1 (25.0) | 0 | 0 | 0 | 1 (16.7) | 3 (50.0) | 2 (33.3) | 7 (17.9) |
| seizure | 1 (50.0) | 1 (25.0) | 0 | 1 (16.7) | 2 (33.3) | 1 (16.7) | 0 | 1 (16.7) | 7 (17.9) |
| alopecia | 0 | 1 (25.0) | 0 | 0 | 1 (16.7) | 1 (16.7) | 2 (33.3) | 1 (16.7) | 6 (15.4) |
| decreased appetite | 0 | 1 (25.0) | 0 | 0 | 1 (16.7) | 2 (33.3) | 1 (16.7) | 1 (16.7) | 6 (15.4) |
| decreased lymphocyte count | 0 | 2 (50.0) | 0 | 0 | 1 (16.7) | 2 (33.3) | 0 | 1 (16.7) | 6 (15.4) |
| decreased neutrophil count | 0 | 2 (50.0) | 2 (66.7) | 1 (16.7) | 0 | 0 | 1 (16.7) | 0 | 6 (15.4) |
| diarrhea | 0 | 0 | 0 | 1 (16.7) | 1 (16.7) | 1 (16.7) | 2 (33.3) | 0 | 5 (12.8) |
| hypertrigly-ceridaemia | 0 | 1 (25.0) | 0 | 1 (16.7) | 1 (16.7) | 1 (16.7) | 1 (16.7) | 0 | 5 (12.8) |
| hyponatremia | 0 | 1 (25.0) | 1 (33.3) | 1 (16.7) | 0 | 0 | 1 (16.7) | 1 (16.7) | 5 (12.8) |
| decreased platelet count | 0 | 2 (50.0) | 1 (33.3) | 0 | 1 (16.7) | 0 | 1 (16.7) | 0 | 5 (12.8) |
| increase in blood cholesterol | 0 | 0 | 0 | 0 | 0 | 1 (16.7) | 2 (33.3) | 1 (16.7) | 4 (10.3) |
| Confusional state | 0 | 0 | 0 | 0 | 1 (16.7) | 1 (16.7) | 0 | 2 (33.3) | 4 (10.3) |
| Gastrointestinal disorder | 0 | 0 | 0 | 0 | 2 (33.3) | 0 | 1 (16.7) | 1 (16.7) | 4 (10.3) |
| Gait disturbance | 0 | 0 | 0 | 0 | 1 (16.7) | 0 | 1 (16.7) | 2 (33.3) | 4 (10.3) |
| Hiccups | 0 | 2 (50.0) | 0 | 0 | 0 | 0 | 0 | 2 (33.3) | 4 (10.3) |
| maculopapular rash | 0 | 0 | 0 | 0 | 0 | 0 | 1 (16.7) | 3 (50.0) | 4 (10.3) |
| Tremor | 0 | 0 | 0 | 0 | 2 (33.3) | 0 | 2 (33.3) | 0 | 4 (10.3) |
| TEAE: Adverse event that emerged or worsened after administration of the study drug. | | | | | | | | | |
| Note: Based on MedDRA ver. 24.1 | | | | | | | | | |
| Source: t_14_3_2_x_ae_by_pt.sas; t_14_3_2_x_ae_by_pt_grd3.sas | | | | | | | | | |
| Data extraction date: April 21, 2024; Data cutoff date: April 21, 2024. | | | | | | | | | |

### Example 4: Preliminary Evaluation of the Antitumor Activity of Compound 1

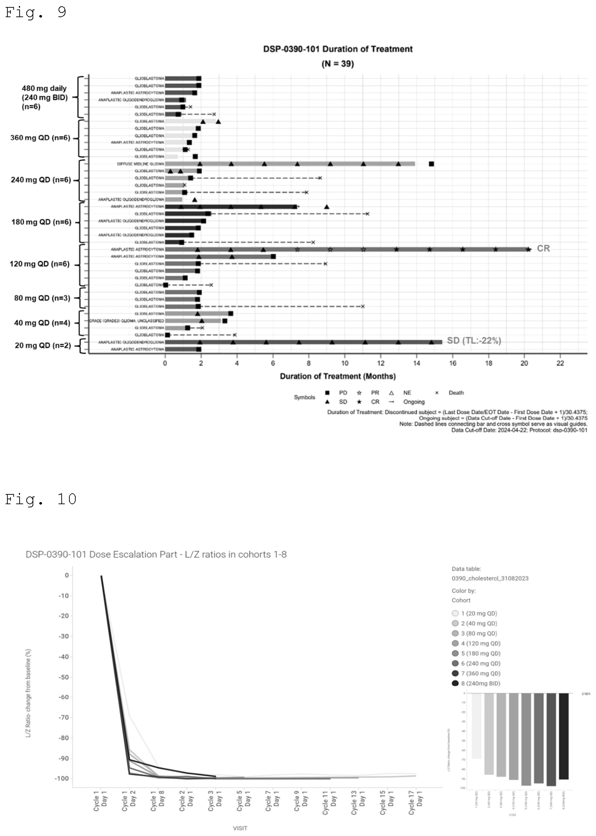

As of the data cutoff date of April 21, 2024, among the 39 patients enrolled in the dose-escalation part, two patients with IDH-mutant grade III astrocytoma showed reductions of 22% and 100% in enhancing lesions based on the RANO criteria and remained on study for 15 and 20 months, respectively (Figure 9).

### Example 5: Pharmacodynamic Assessment of Compound 1

As of April 2024, pharmacodynamic evaluation of the 39 patients enrolled in the dose-escalation part demonstrated that Compound 1 inhibited the target, at least in serum.

Across the entire cohort, the mean L/Z ratio decreased rapidly after administration of Compound 1. By Cycle 1 Day 2 (C1D2), the L/Z ratio decreased by 70% at 20 mg QD, 85-91% at 40-120 mg QD, 95-97% at 180 mg and 240 mg QD, and 97.5% at 360 mg QD.

By Cycle 1 Day 8 (C1D8), the L/Z ratio decreased by approximately 100% across the dose range of 20-360 mg QD, and this level was maintained in subsequent cycles. A strong dose-dependent effect was observed between 20 mg and 40 mg, whereas the change was more gradual between 40 mg and 360 mg. The data for 180 mg QD, 240 mg QD, and 360 mg QD were comparable. Patients receiving 480 mg per day (240 mg BID) did not show a greater reduction in the L/Z ratio than those at the 180-360 mg QD dose levels (Figure 10).

### Example 6: Pharmacokinetic Assessment of Compound 1 (1)

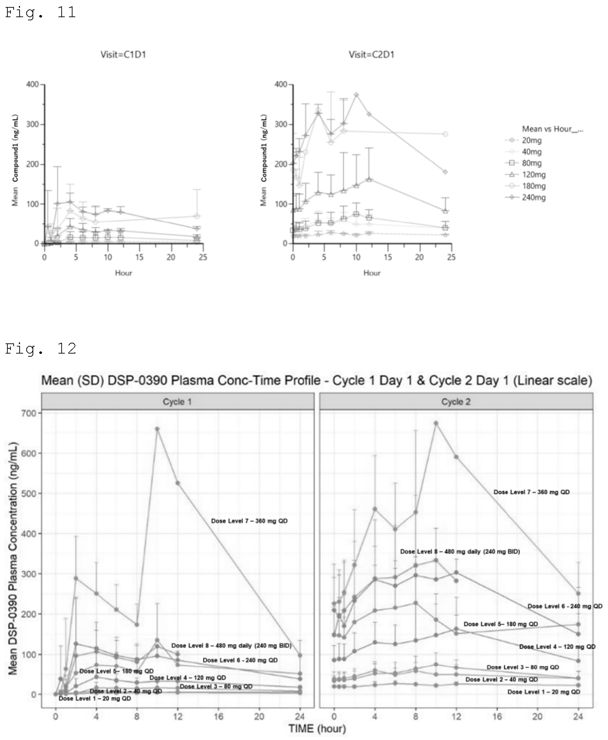

Summary statistics based on preliminary data were obtained for the pharmacokinetics of Compound 1 in patients receiving once-daily doses ranging from 20 mg to 240 mg (Table 5). The mean plasma concentration-time curves of Compound 1 in the once-daily dose groups ranging from 20 mg to 240 mg are shown in Figure 11. In this study, one cycle consists of 28 days, and Cycle 1 Day 1 refers to Day 1 of the first cycle. In all dose groups, the geometric mean of Cmax on Cycle 1 Day 1 ranged from 4 to 145 ng/mL, the geometric mean of AUC₀₋₂₄ ranged from 76 to 1482 ng·h/mL, and the median Tmax ranged from 2 to 8 hours. On Cycle 2 Day 1, the geometric mean of Cmax ranged from 27.7 to 359 ng/mL, the geometric mean of AUC₀₋₂₄ ranged from 556.5 to 6671 ng·h/mL, and the median Tmax ranged from 2 to 7 hours. Accumulation was observed after repeated administration. The mean accumulation ratios for Cmax and AUC₀₋₂₄ were 2.6-6.2 and 3.9-8.6, respectively. Based on these accumulation ratios, the half-life of Compound 1 was estimated to be approximately 4 days. Based on the currently available data, plasma concentrations are considered to have reached steady state by Day 15. On Cycle 1 Day 1 and Cycle 2 Day 1, the exposure (Cmax and AUC₀₋₂₄) of Compound 1 increased in a dose-dependent manner (data cut-off: April 2023).

In the table, AR denotes the accumulation ratio, and NC indicates not calculated.

**[Table 5]**

| | | Cycle 1 Day 1 | | | Cycle 2 Day 1 | | | AR | |
|---|---|---|---|---|---|---|---|---|---|
| | | Tₘₐₓ (h) | Cₘₐₓ (ng/ mL) | AUC₀₋₂₄ (ng*h / mL) | Tₘₐₓ (h) | Cₘₐₓ (ng/ mL) | AUC₀₋₂₄ (ng*h / mL) | AR Cₘₐₓ | AR AUC |
| Cohort 1 20 mg, QD | N | 2 | 2 | 2 | 2 | 2 | 2 | 2 | 2 |
| | mean | 6 | 4 | 77 | 7 | 28 | 561 | 6.1 | 7.4 |
| | median | 6 | 4 | 77 | 7 | 28 | 561 | NC | NC |
| | geometric mean | NC | 4 | 76 | NC | 28 | 557 | NC | NC |
| | geometric coefficient of variation (%) | NC | 23 | 26 | NC | 18 | 17 | NC | NC |
| Cohort | N | 4 | 4 | 4 | 3 | 3 | 3 | 3 | 3 |
| 2 40 mg, QD | mean | 11 | 10 | 132 | 5 | 60 | 1175 | 6.2 | 8. 6 |
| | median | 8 | 7 | 107 | 4 | 72 | 1367 | NC | NC |
| | geometric mean | NC | 8 | 123 | NC | 56 | 1106 | NC | NC |
| | geometric coefficient of variation (%) | NC | 73 | 42 | NC | 47 | 47 | NC | NC |
| Cohort 3 80 mg, QD | N | 3 | 3 | 3 | 3 | 3 | 3 | 3 | 3 |
| | mean | 8 | 17 | 268 | 8 | 63 | 1186 | 5.8 | 6.9 |
| | median | 8 | 15 | 205 | 8 | 61 | 1112 | NC | NC |
| | geometric mean | NC | 13 | 203 | NC | 58 | 1099 | NC | NC |
| | geometric coefficient of variation (%) | NC | 123 | 119 | NC | 59 | 51 | NC | NC |
| Cohort 4 120 mg, QD | N | 6 | 6 | 6 | 4 | 4 | 4 | 4 | 4 |
| | mean | 4 | 44 | 583 | 6 | 156 | 2632 | 4.6 | 5.3 |
| | median | 4 | 45 | 605 | 5 | 138 | 2326 | NC | NC |
| | geometric mean | NC | 40 | 544 | NA | 140 | 2422 | NC | NC |
| | geometric coefficient of variation (%) | NC | 59 | 45 | NA | 59 | 49 | NC | NC |
| Cohort 5 180 mg, QD | N | 3 | 3 | 3 | 2 | 2 | 1 | 2 | 1 |
| | mean | 11 | 101 | 1422 | 7 | 287 | 7260 | 2 | 3.9 |
| | median | 6 | 140 | 1840 | 7 | 287 | 7260 | NC | NC |
| | geometric mean | NC | 73 | 1059 | NC | 281 | 7260 | NC | NC |
| | geometric coefficient of variation (%) | NC | 168 | 150 | NC | 30 | | NC | NC |
| Cohort 6 240 mg, QD | N | 5 | 5 | 4 | 2 | 2 | 1 | 2 | 1 |
| | mean | 3 | 153 | 1509 | 7 | 360 | 6671 | 2.6 | 3.9 |
| | median | 2 | 148 | 1624 | 7 | 360 | 6671 | NC | NC |
| | geometric mean | NC | 145 | 1482 | NC | 359 | 6671 | NC | NC |
| | geometric coefficient of variation (%) | NC | 37 | 23 | NC | 6 | | NC | NC |

### Example 7: Pharmacokinetic Assessment of Compound 1 (2)

Summary statistics based on preliminary data were obtained for the pharmacokinetics of Compound 1 in patients in the 20 mg QD, 40 mg QD, 80 mg QD, 120 mg QD, 180 mg QD, 240 mg QD, 360 mg QD, and 480 mg daily (240 mg BID) dose groups (Table 6). The mean plasma concentration-time curves of Compound 1 in the 20 mg QD to 480 mg daily (240 mg BID) dose groups are shown in Figure 12 (in Figure 12, Compound 1 is denoted as "DSP-0390"). In this study, one cycle consists of 28 days, and Cycle 1 Day 1 refers to Day 1 of the first cycle. Preliminary pharmacokinetic data showed that the exposure of Compound 1 increased in a dose-dependent manner over the range of 20 mg to 360 mg QD. The median Tmax ranged from 2 to 8 hours. Accumulation was observed after repeated administration, and assuming linearity based on the observed accumulation ratios, the estimated elimination half-life was considered to be on the order of several days (approximately 1 to 6 days) (data cut-off: March 2024).

**[Table 6]**

| | **Cycle 1, Day 1** | | | | **Cycle 2, Day 1** | | | | | | |
|---|---|---|---|---|---|---|---|---|---|---|---|
| | **N** | **Tmax (hr) [a]** | **Cmax (n g/mL)** | **AUCtau (ng *h/ml) [b]** | **N** | **Tmax,ss (h) [a]** | **Cmax,ss (ng/ml)** | **AUCtau,ss ( ng*h/ml) [b]** | **AR Cmax** | **AR AUC** | **Effective T1/2 (days)** |
| **Cohort 1 20 mg QD** | 2 | 6 | 4.28 | 76.6 | 2 | 7 | 27.9 | **560** | 6.52 | 7.31 | 4.7 |
| **Cohort 2 40 mg QD** | 4 | 8 | 9.87 | 131 | 3 | 4 | 59.6 | 1170 | 6.04 | 8.93 | 5.8 |
| **Cohort 3 80 mg QD** | 3 | 8 | 17.1 | 264 | 3 | 8 | 63.2 | 1180 | 3.70 | 4.47 | 2.7 |
| **Cohort 4 120 mg QD** | 6 | 4 | 44.2 | 577 | 4 | 5 | 156 | 2610 | 3.53 | 4.52 | 2.8 |
| **Cohort 5 180 mg QD** | 6 | 6 | 114 | 1370 | 5 | 6 | 215 | 4860(n=3) | 1.89 | 3.55 | 2.1 |
| **Cohort 6 240 mg QD** | 6 | 2 | 177 | 1620 | 4 | 5 | 316 | 5330 | 1.79 | 3.29 | 1.9 |
| **Cohort 7 360 mg QD** | 6 | 4 | 359 | 4110 | 4 | 4 | 489 | 8490 | 1.36 | 2.07 | 1.0 |
| **Cohort 8 480 mg (240 mg BID)** | 6 | 4 | 143 | 1980(n=1) | 4 | 7 | 346 | 3180(n=1) | 2.42 | 1.61 | 0.7 |

| | | | | | | | | | | | |
|---|---|---|---|---|---|---|---|---|---|---|---|
| [a] median value [b] AUCtau: area under the plasma concentration-time curve over the dosing interval (tau) (tau = 24 hours for QD and 12 hours for BID); AR: accumulation ratio | | | | | | | | | | | |

### Example 8: Determination of the Recommended Dose for Expansion (RDE)

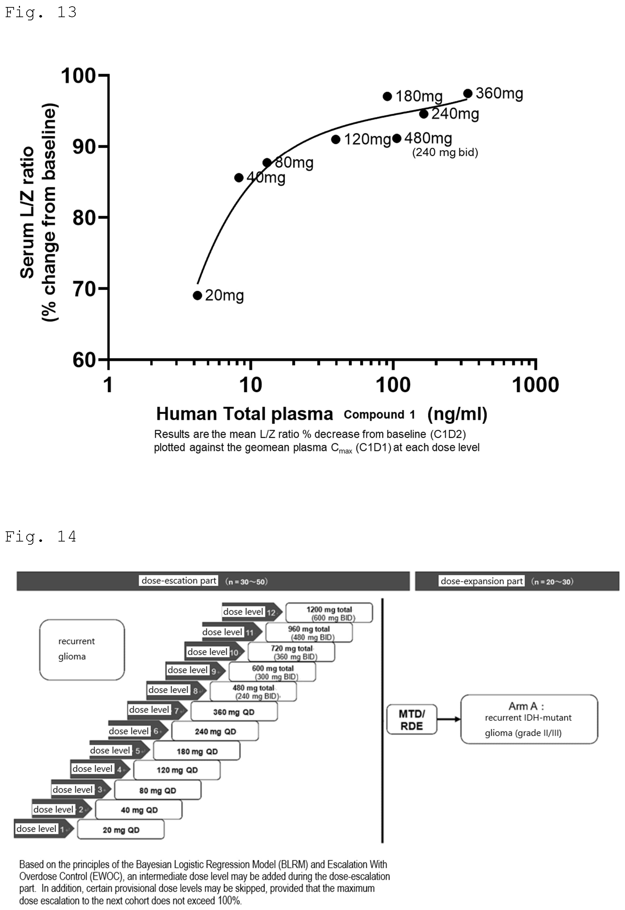

As described in Example 3, the MTD of Compound 1 was determined to be 480 mg daily (240 mg BID). In addition, with increasing dose, Grade 2 or 3 adverse events related to Compound 1 were observed more frequently, particularly in dose groups of 240 mg QD or higher than in dose groups of 180 mg or lower, thereby suggesting dose-dependent toxicity. In the evaluation of PK/PD data, the exposure of Compound 1 increased in a dose-dependent manner over the range of 20 mg QD to 360 mg QD. In contrast, the blood L/Z ratio remained at a similar level from the 180 mg QD to 360 mg QD dose groups, thereby suggesting that a plateau may have been reached (Figure 13). In the preliminary evaluation of antitumor activity, tumor shrinkage was observed at 20 mg QD and 120 mg QD (22% and 100% reduction from baseline at 20 mg QD and 120 mg QD, respectively), and prolonged stable disease (SD) was observed at 180 mg QD and 240 mg QD. In contrast, at 360 mg QD and 480 mg daily (240 mg BID), most patients discontinued administration of Compound 1 within two months due to objective disease progression according to RANO criteria. These findings indicate that the clinical activity of Compound 1 was observed in dose groups of 240 mg or lower, and no dose-dependent increase in antitumor activity was observed. Based on all available clinical data, and as a result of discussions between all investigators participating in the clinical study and the sponsor, 180 mg QD was determined as the recommended dose for expansion (RDE), as a dose that is tolerable over the long term and expected to provide efficacy.

### 2. Clinical Trial in Patients with Recurrent Malignant Glioma Classified as WHO grade II, III or IV

### (1) Clinical trial outline

The present study is a Phase 1 dose-escalation trial with a dose-expansion part designed to evaluate the safety, pharmacokinetics (PK), pharmacodynamics (PD), and preliminary antitumor activity of Compound 1 following oral administration in patients with recurrent malignant glioma classified as WHO grade II, III or IV. In the present study, Compound 1 is orally administered daily. Each treatment cycle is defined as 28 days, during which safety and treatment response are evaluated. Administration to each subject is continued until unacceptable toxicity occurs, consent is withdrawn, the subject is lost to follow-up, or discontinuation is determined by the investigator and/or the sponsor.

An overview of the overall study design is shown in Figure 14. The present study consists of two parts: a dose-escalation part (Part 1) and a dose-expansion part (Part 2). In the dose-escalation part, the dose of Compound 1 is escalated in patients with malignant glioma classified as WHO grade II, III or IV to determine the maximum tolerated dose (MTD) and/or the recommended dose for the dose-expansion part (RDE), which may be lower than the MTD. Once the MTD and/or RDE is established, a dose-expansion part in patients with non-enhancing glioma with an IDH mutation classified as WHO grade II or III is initiated to evaluate the preliminary clinical activity, as well as the safety and tolerability, of Compound 1.

Approximately 30-50 subjects are enrolled in the dose-escalation part of this study. In addition, approximately 20-30 subjects are enrolled in the dose-expansion part. Since the number of subjects in each cohort may vary dynamically in a Bayesian adaptive design, the exact number of subjects cannot be specified in advance.

### (2) Overall study design

The initial starting dose of 20 mg/day (expressed as the present compound) was determined in accordance with the ICH S9 guideline of the International Council for Harmonisation of Technical Requirements for Pharmaceuticals for Human Use (ICH). Based on a 4-week mouse toxicity study conducted in compliance with Good Laboratory Practice (GLP), the dose causing severe toxicity in 10% of the test animals (STD10) was determined to be 50 mg/kg. Applying a safety factor of 10 and interspecies scaling, the starting dose for this first-in-human (FIH) study was calculated to be 0.4 mg/kg, corresponding to a fixed dose of 24 mg for a 60-kg human. In a 4-week non-human primate toxicity study conducted in compliance with Good Laboratory Practice (GLP), the highest non-severely toxic dose (HNSTD) was determined to be 7.5 mg/kg. Applying a safety factor of 6 and interspecies scaling, the starting dose in humans was estimated to be 0.4 mg/kg, corresponding to a fixed dose of 24 mg. The final recommended starting dose for the first-in-human (FIH) study was rounded to 20 mg/day. This recommended starting dose exceeds the minimum pharmacologically active dose in humans of 15 mg, which corresponds to the human equivalent dose (HED) of 3 mg/kg at which a consistent tumor shrinkage effect was observed in mice bearing U-87 GBM xenografts.

The provisional dose levels are shown in Table 7.

**[Table 7]**

| Dose level | Dose |
|---|---|
| 1 | 20 mg QD |
| 2 | 40 mg QD |
| 3 | 80 mg QD |
| 4 | 120 mg QD |
| 5 | 180 mg QD |
| 6 | 240 mg QD |
| 7 | 360 mg QD |
| 8 | A total of 480 mg (240 mg BID) |
| 9 | A total of 600 mg (300 mg BID) |
| 10 | A total of 720 mg (360 mg BID) |
| 11 | A total of 960 mg (480 mg BID) |
| 12 | A total of 1200 mg (600 mg BID) |
| Note: Based on the principles of the Bayesian Logistic Regression Model (BLRM) and Escalation With Overdose Control (EWOC), an intermediate dose level may be added during the dose-escalation part. In addition, certain provisional dose levels may be skipped, provided that the maximum dose escalation to the next cohort does not exceed 100%. | |

An alternative dosing schedule, such as twice-daily (BID) administration, is initiated at a total daily dose of 480 mg in order to reduce pill burden (see the table of provisional dose levels for details). A change in the dosing schedule from once daily (QD) to twice daily (BID) is not expected to affect the steady-state mean exposure at the same total daily dose.

### (3) Dose-escalation part

The maximum tolerated dose (MTD) is estimated based on the occurrence of dose-limiting toxicities (DLTs) during the DLT evaluation period (Cycle 1; 28 days from the start of investigational drug administration), using an adaptive Bayesian logistic regression model (BLRM, Section 20.1) with escalation with overdose control (EWOC) as the guideline for dose escalation. Each dose level is evaluated in cohorts of 1-6 patients. In all cohorts, administration of the investigational drug to the second and subsequent patients is permitted after completion of all safety assessments on Day 8 of Cycle 1 in the first patient. If a DLT is observed at a given dose level, at least three patients are evaluated at that dose level. At dose level 2 in the dose-escalation part, at least three patients are required to be evaluated before escalation to a higher dose level. After completion of each cohort, the probability of observing a DLT at each dose level in the present study is updated using the BLRM based on cumulative data from the preceding cohorts, and the MTD is estimated.

The safety at each dose level is evaluated at a Dose Determination Meeting (DDM) composed of the sponsor's representatives (the medical monitor serving as chair of the meeting, the clinical development lead, the lead statistician, and representatives from pharmacovigilance, clinical pharmacology, and biomarker functions), as well as the principal investigators participating in the present study. If necessary, consultation with external experts (e.g., ophthalmologists) may also be sought. The primary objective of the DDM is to determine the dose and dosing schedule, as well as the number of additional patients to be treated with the investigational drug in the present study. Representatives from all study sites (preferably including the principal investigator) will be invited to participate in the DDM; however, participation will be required only for sites with patients currently receiving the investigational drug. At the conclusion of the DDM, the medical monitor, the representative from pharmacovigilance function, and the principal investigators reach agreement on the subsequent dosing and enrollment decisions in accordance with the study protocol. To convene a DDM, the required number of patients must have completed the minimum evaluation period in accordance with the study protocol, or a predefined number of DLTs must have occurred.

The decision to escalate, de-escalate, or maintain the dose in the next cohort is made based on the recommendation from the BLRM and a review of other available data, including PK and PD data from all patients who receive the investigational drug; patient characteristics, medical history, and prior anticancer therapies; investigational drug administration data such as dose reductions or discontinuations; clinical laboratory results; imaging findings and performance status assessments; adverse events (AEs) and serious adverse events (SAEs); and other clinical data, including whether DLTs have occurred.

Based on the Bayesian logistic regression model (BLRM) and the principle of escalation with overdose control (EWOC), intermediate dose levels may be added during the dose-escalation part.

If a given dose level is reviewed and considered safe, additional patients may be enrolled in an enrichment cohort at that dose level to obtain further data to evaluate its safety and tolerability, preliminary antitumor activity, and PK and PD. In the enrichment cohort, a one-week waiting period between the first patient and the subsequent patient is not required.

Intra-patient dose escalation is optional and can be implemented at the discretion of the principal investigator, etc. if a higher dose had been shown to be safe and the patient was considered likely to benefit from dose escalation. Intra-patient dose escalation is not permitted at any time within the first two cycles at the initially assigned dose level. After completion of Cycle 2, administration of Compound 1 at a higher dose than the initially assigned dose may be considered for individual patients. Before a patient could receive Compound 1 at a higher dose, tolerability at the lower dose has to be confirmed during at least two cycles of treatment (*i.e*., no Compound 1-related non-hematologic or hematologic toxicities of CTCAE Grade ≥2 occur at the initially assigned lower dose). Furthermore, the new higher dose to be administered to the patient has to be a dose level that has already been evaluated and is not above the MTD. There is no limit on the number of times the dose of Compound 1 could be escalated for an individual patient.

The following rules are applied to any further dose escalations after the first intra-patient dose escalation. That is, the patient must not experience any Compound 1-related non-hematologic or hematologic toxicities of CTCAE Grade ≥2 at the lower dose during at least the two most recent treatment cycles. In addition, the higher dose under consideration has to have been adequately evaluated and demonstrated not to exceed the MTD.

### (4) Dose-expansion part

Once the MTD and/or RDE is established, the dose-expansion part of the study is initiated. In the dose-expansion part, approximately 20-30 patients with recurrent non-enhancing IDH-mutant glioma (WHO grade II or III) are enrolled to evaluate the preliminary antitumor activity of Compound 1 administered at the selected dose and to further assess its safety and tolerability. A Bayesian approach may be used to evaluate the posterior probability of achieving an ORR [complete response (CR), partial response (PR), or minor response (MR)] based on RANO-LGG criteria.

In the dose-expansion part, the DLT rate is monitored using the BLRM model and escalation with overdose control (EWOC). If one or more DLTs are observed during dose expansion, the BLRM model is fitted at the time of the first DLT occurrence. Dose expansion is discontinued only if a lower dose is recommended by the BLRM model and the sponsor and principal investigators jointly determine, based on a review of the model estimates and the available safety, PK, PD, and other clinical data, that dose expansion should be stopped. If additional DLTs occur thereafter, the BLRM model is refitted, and a decision on whether to discontinue the dose-expansion part is made in accordance with the procedure described above after the first DLT occurrence.

In addition, in the dose-expansion part initiated after establishment of the MTD and/or RDE, patient enrollment is restricted to those with specific genetic alterations. Such specific genetic alterations refer to genetic abnormalities for which Compound 1 is expected to be effective, such as IDH mutations. Recently, in a phase 3 trial of vorasidenib, a dual inhibitor of mutant IDH1 and IDH2 enzymes, progression-free survival was prolonged compared with placebo in patients with grade 2 IDH-mutant glioma for whom an active surveillance strategy was considered appropriate, and the initiation of subsequent anticancer therapy was delayed. In addition, emerging evidence suggests a link between IDH-mutant low-grade glioma and cholesterol metabolism. A recent study reported that IDH mutations in glioma increased 24(S)-hydroxycholesterol (24-OHC), resulting in reduced cholesterol levels and activation of liver X receptor (LXR). As a result, the sensitivity of IDH1-mutant glioma cells to atorvastatin, an inhibitor of 3-hydroxy-3-methylglutaryl-CoA reductase, is increased. Furthermore, recent bioinformatics analyses have suggested that cholesterol metabolism plays an important role in immunosuppression in low-grade glioma and serves as an independent prognostic factor for these tumors. Thus, regulation of cholesterol biosynthesis in IDH-mutant glioma may represent a potential therapeutic target.

### (5) Description of dose-escalation and dose-expansion parts

Compound 1 is supplied as 20 mg and 60 mg capsules. Compound 1 is administered orally daily preferably with approximately 200 mL of water or about half a glass of water. Compound 1 is administered after a fasting period of at least 6 hours, and patients are required to remain fasting for 1 hour after dosing. Alternative dosing regimens (e.g., BID) may be studied. For BID dosing, the morning dose of Compound 1 is administered after a fasting period of at least 6 hours, and patients are required to remain fasting for 1 hour after dosing. For the evening dose, Compound 1 is administered after a fasting period of 2 hours, and patients are required to remain fasting for 1 hour after dosing. The evening dose is administered approximately 12 hours after the morning dose of Compound 1.

In the dose-escalation part, patients continue treatment at the assigned dose until the discontinuation criteria are met. Based on the BLRM recommendation and a review of safety data, intermediate dose levels may be explored instead of the dose levels described as provisional dose levels. In addition, certain provisional dose levels may be skipped, provided that the maximum dose-escalation increment to the next cohort does not exceed 100%.

Patient compliance with dosing is monitored at the time points specified by the sponsor based on patient dosing diaries and/or investigational drug accountability records. Patients record the administration of Compound 1 using a patient dosing diary.

In the dose-expansion part, the recommended dose for expansion is determined based on the overall data obtained in the dose-escalation part, including safety, PK, PD, and preliminary clinical activity. At the end of the dose-expansion part, enrollment of additional patients at one or more dose levels may be considered for dose optimization based on the safety, clinical activity, PK, and PD data collected during this study.

### Example 9: In vivo Efficacy Study of Combination Therapy with Bevacizumab

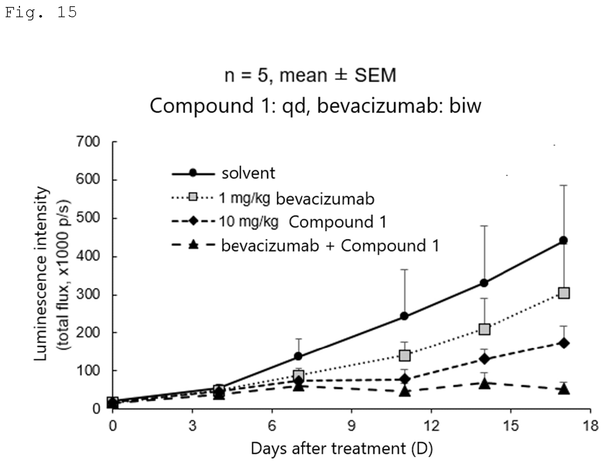

Luciferase-expressing U-87 cells, in which the luminescent enzyme luciferase was forcibly expressed, were established (obtained from ATCC (American Type Culture Collection)). 1 x 10⁶ of these cells were implanted into the brains of immunodeficient mice to establish brain tumors. On day 18 after tumor cell implantation, administration was initiated with Compound 1 at 10 mg/kg (twice daily) and bevacizumab at 1 mg/kg (twice weekly). On day 30, the dosing frequency of Compound 1 was reduced to once daily. The efficacy of each monotherapy and the combination therapy was evaluated by quantifying orthotopic tumor progression using an IVIS Imaging System and comparing the results. The results are shown in Figure 15.

Compound 1 was found to exhibit a remarkably enhanced antitumor effect when used in combination with bevacizumab.

### Example 10: In vivo Efficacy Study of Combination Therapy with Ipilimumab

MC38 cells (3 x 10⁵) were subcutaneously implanted into C57BL/6J mice (n = 6) to establish tumors. On day 12 after implantation, administration was initiated with Compound 1 at 3 mg/kg or 30 mg/kg (once daily) and ipilimumab (an anti-CTLA-4 antibody) at 5 mg/kg (once weekly), and the efficacy of each monotherapy and the combination therapy was compared.

B16 cells (3 x 10⁵) were subcutaneously implanted into C57BL/6J mice (n = 6) to establish tumors. On day 12 after implantation, administration was initiated with Compound 1 at 3 mg/kg or 10 mg/kg (once daily) and ipilimumab (an anti-CTLA-4 antibody) at 5 mg/kg (once weekly), and the efficacy of each monotherapy and the combination therapy was compared.

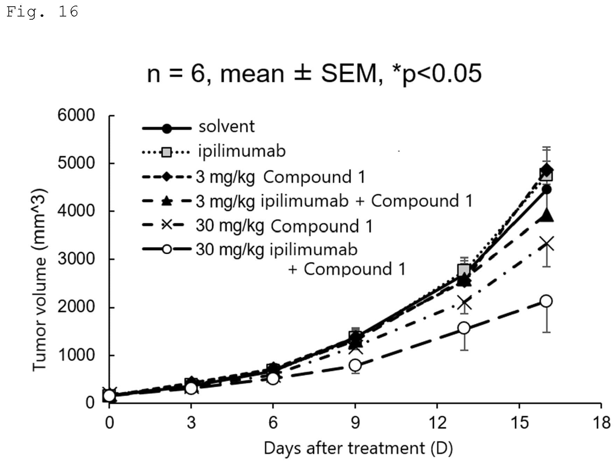

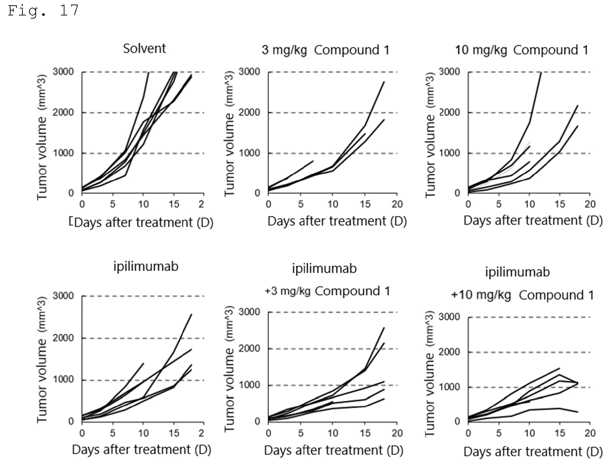

The results obtained in mice implanted with MC38 cells are shown in Figure 16, and the results obtained in mice implanted with B16 cells are shown in Figure 17.

Tumor volume was calculated using the following formula based on the short and long diameters of the tumor measured with digital calipers (Mitutoyo). 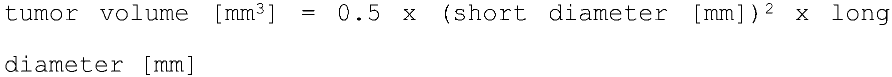 tumor volume [mm3] = 0.5 x (short diameter [mm])2 x long diameter [mm]

The antitumor effect was evaluated by comparing the vehicle-treated control group with the test substance-treated group, and calculating T/C using the following formula. 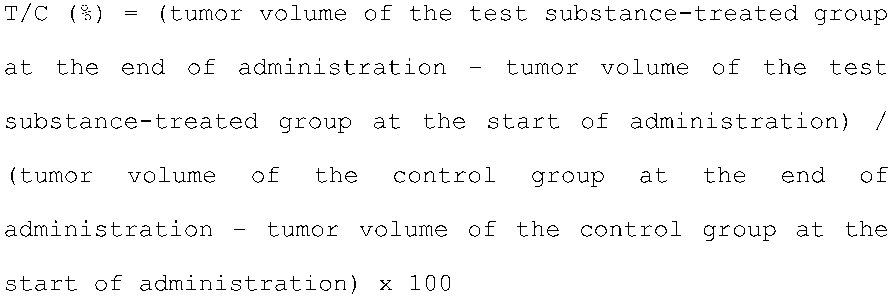 T/C (%) = (tumor volume of the test substance-treated group at the end of administration - tumor volume of the test substance-treated group at the start of administration) / (tumor volume of the control group at the end of administration - tumor volume of the control group at the start of administration) x 100

In a combination efficacy study using mice implanted with MC38 cells, Compound 1 exhibited a remarkably pronounced antitumor effect when administered in combination with ipilimumab. Furthermore, Compound 1 exhibited more pronounced efficacy at higher doses, indicating a dose-dependent synergistic effect.

In a combination efficacy study using mice implanted with B16 cells, Compound 1 exhibited a remarkably pronounced antitumor effect when administered in combination with ipilimumab. Furthermore, Compound 1 exhibited more pronounced efficacy at higher doses, indicating a dose-dependent synergistic effect. This tumor-bearing mouse model is known to be resistant to ipilimumab, suggesting that Compound 1 may contribute to overcoming this resistance.

Compound 1 demonstrated acceptable safety at doses of 20 - 240 mg once daily (QD) (Example 1), and clinical results suggesting efficacy were obtained at doses of 20 - 240 mg QD (Example 2). Thus, safety and efficacy may be maintained even at doses of 240 mg QD or higher. Based on the results of additional studies, Compound 1 demonstrated acceptable safety and efficacy at doses ranging from 20 mg to 360 mg QD (Examples 3 and 4). Furthermore, based on all available clinical data, 180 mg QD was determined as the recommended dose for expansion (RDE), as it is expected to be tolerable over the long term and to provide efficacy (Example 8). Furthermore, Compound 1 was found to exhibit a remarkably pronounced antitumor effect when used in combination with bevacizumab (Example 9). In addition, Compound 1 was also confirmed to exhibit a particularly pronounced antitumor effect when used in combination with ipilimumab (Example 10). Notably, in B16 tumor-bearing mice - an ipilimumab-resistant model - Compound 1 exhibited a pronounced antitumor effect in combination with ipilimumab, confirming its contribution to overcoming this resistance (Example 10).

### Example 11: Efficacy Evaluation in an Anti-PD-1 Antibody-Resistant in vivo Model

B16 cells (5 x 10⁵ cells; obtained from the JCRB Cell Bank) were intradermally implanted into C57BL/6J mice to establish tumors. On day 12 after implantation, administration of Compound 1 (10 mg/kg, once daily) or an anti-PD-1 antibody (nivolumab, 5 mg/kg, once weekly) was initiated, and the efficacy of each monotherapy group was compared with that of the control group.

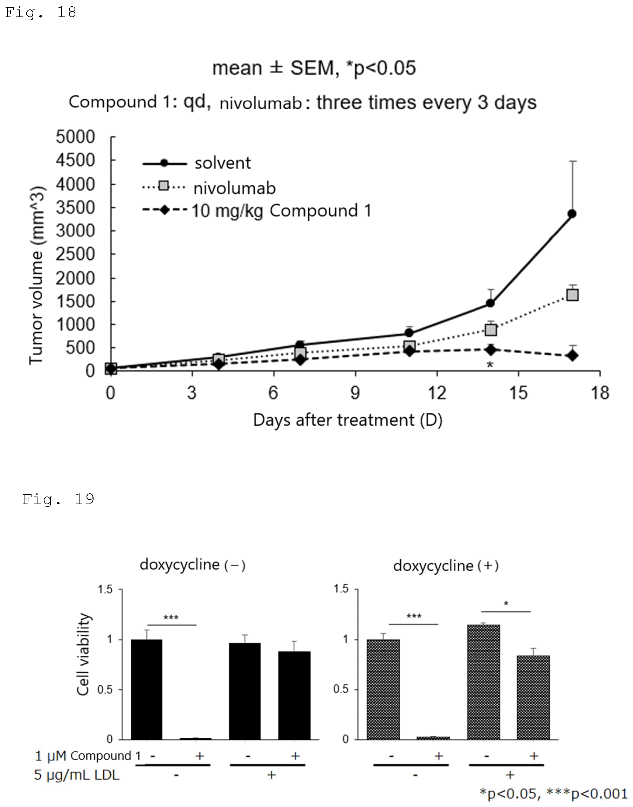

The Compound 1-treated group showed a statistically significant inhibition of tumor growth compared with the nivolumab-treated group in the B16 tumor-bearing model, an anti-PD-1 antibody-resistant cancer model (Figure 18). In other words, Compound 1 demonstrated tumor growth inhibitory effects as a monotherapy against cancers resistant to anti-PD-1 antibody therapy.

### Example 12: Cytotoxicity assay in IDH1 R132H mutant U-87 cells under in vitro sphere culture conditions with low-density lipoprotein (LDL) supplementation

In brain tumor tissues, peripheral LDL is generally considered unable to cross the blood-brain barrier, and brain tumor cells are thought to proliferate in a manner dependent on endogenous cholesterol biosynthesis pathways. However, in recent years, it has been reported that the integrity of the blood-brain barrier is not maintained due to glioma progression as well as tissue damage caused by standard treatments such as surgery, radiotherapy, and chemotherapy. In other words, disruption of the blood-brain barrier may allow peripheral LDL to enter brain tissue, potentially leading to increased LDL levels within gliomas.

The present compound exerts cytotoxic effects on cancer cells in a manner dependent on the intracellular cholesterol biosynthesis pathway. On the other hand, it has been reported that when low-density lipoprotein (LDL) is added to cells and intracellular LDL levels increase, cells become less dependent on the intracellular cholesterol biosynthesis pathway (PLoS One. 2012; 7(6): e39445). Since cholesterol is supplied via an alternative pathway, the cytotoxicity of the present compound due to EBP inhibition is considered to be markedly reduced.

It has been reported that the IDH1 R132H mutation reduces the expression level of the LDL receptor (Oncogene (2020) 39:6340-6353). In other words, even when LDL is added to the cells, intracellular LDL levels in cells harboring this mutation remain lower than those in wild-type cells. Therefore, the contribution of cholesterol supply via the intracellular cholesterol biosynthesis pathway remains substantial, and the present compound is expected to exert stronger cytotoxic effects via EBP inhibition in the mutant cells.

U-87 cells were established with a tetracycline-inducible IDH1 R132H mutation expression system. The cells were cultured in the presence of doxycycline (1 µM), a tetracycline derivative, for 5 days to induce the IDH1 R132H mutation. After induction of the mutation, the cells were transferred to sphere culture medium (containing B-27 supplement, epidermal growth factor, cell proliferation factors, insulin, and low-density lipoprotein [LDL]), and cultured for 7 days under sphere-forming conditions after the addition of Compound 1 (1 µM). The LDL concentration in the medium was 5 µg/mL. After incubation, cellular ATP levels were quantified as an indicator of cell viability, and cell viability was calculated as the ratio relative to ATP levels under vehicle-only conditions. Using this approach, the cytotoxic effects of the present compound under LDL-supplemented conditions were compared between wild-type (doxycycline-) and mutant (doxycycline+) cells.

Under LDL-supplemented conditions, the cytotoxicity of Compound 1 was evaluated in IDH1 wild-type cells and in IDH1 R132H mutant cells, which have lower intracellular LDL levels compared with the wild-type. As shown in Figure 19, under conditions of high LDL supplementation, Compound 1 exhibited statistically-significantly stronger cytotoxicity in IDH1 R132H mutant cells compared with wild-type cells.

### Example 13: Evaluation of Cytotoxicity in U-87 Cells (IDH1 wild-type) under in vitro Sphere Culture Conditions with Low-density Lipoprotein (LDL) Supplementation, using Compound 1 in Combination with Ezetimibe

U-87 cells were cultured for 7 days in sphere medium supplemented with LDL at a concentration of 5 µg/mL, with either ezetimibe (a cholesterol absorption inhibitor) added at 3.3, 10, or 30 µM, Compound 1 added at 1 µM, or a combination of both agents. Cell viability was calculated in the same manner as in Example 12, and the cytotoxic effects of each treatment were compared between single-agent and combination conditions.

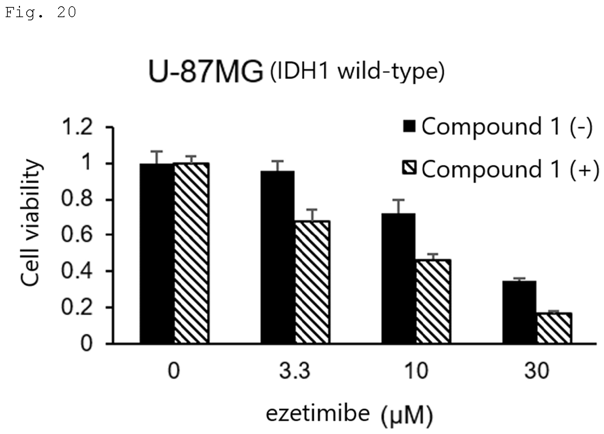

Based on Example 13, the cytotoxicity of the combination of Compound 1 and ezetimibe was evaluated in U-87 cells (IDH1 wild-type) under conditions of high LDL supplementation. As shown in Figure 20, although each agent alone exhibited low cytotoxicity in IDH1 wild-type cells under high LDL conditions, the combination of Compound 1 and ezetimibe exhibited potent cytotoxic effects.

The present compound exhibits highly pronounced cytotoxicity in cell culture systems that depend on the intracellular cholesterol biosynthesis pathway; however, as described above, its activity may be affected by increased LDL levels resulting from disruption of the blood-brain barrier. In gliomas that are less susceptible to the effects of increased LDL levels, however, such as those harboring the IDH1 R132H mutation, sufficient cytotoxicity is expected to be exerted (Example 12). Furthermore, even in gliomas with elevated LDL levels, the present compound is expected to exhibit antitumor effects by demonstrating strong cytotoxicity when used in combination with a cholesterol absorption inhibitor (Example 13).

### Example 14: Cytotoxicity Assay under in vitro Sphere Culture Conditions Using the Active Metabolite

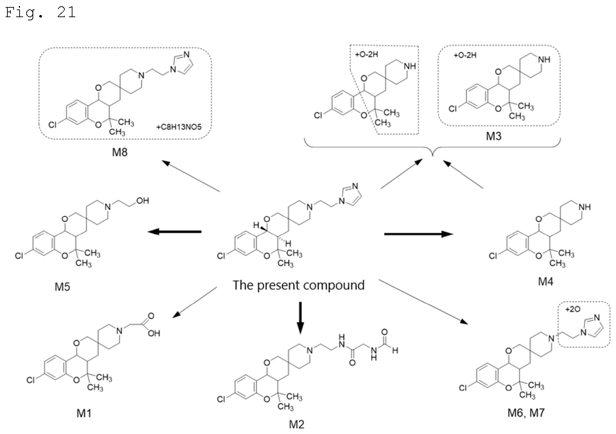

Investigation of the major metabolic pathways of the present compound revealed that, *in vitro* (mouse, rabbit, monkey, and human), the pathways were oxidative metabolism of the imidazole ring (M2), N-dealkylation (M4), and hydroxylation (M5), whereas *in vivo* (mouse and monkey), the pathways were oxidative metabolism of the imidazole ring (M2) and N-dealkylation (M4). Other metabolic pathways included oxidative cleavage and carboxylation of the imidazole ring (M1), N-dealkylation and oxidation (M3), oxidative metabolism at the N-ethyl-imidazole moiety (M6 and M7), and conjugation with N-acetylglucosamine (M8) (Figure 21). Metabolites detected in human substrates were also observed in animal hepatocytes, and no human-specific metabolites were identified. There were no major species differences in the metabolite profile of the present compound.

Based on the following metabolite identification studies, the structures of the metabolites (M2, M4, and M5) detected in mouse and monkey plasma were confirmed by comparison with the synthesized reference standards of Reference examples 18, 9, and 19. The LC/MS/MS analysis conditions used for metabolite identification were as follows.

### LC/MS/MS system:

Detector: API5500 Qtrap LC/MS/MS (Sciex)
Detection mode: MRM mode, Positive ion detection mode
HPLC: Nexera X2 (Shimadzu)
Column: Cadenza CD-C18 (3 µm, 2.0 mm I.D. x 50 mm)
Flow rate: 0.4 mL/min
Condition 1 (for measurement of Compound 1, metabolites M2 and M4)
Mobile phase: A: 10 mM aqueous ammonium acetate, B: methanol
Gradient condition: 0.0-0.3 min B 40%, 0.3-1.7 min B 40% to 90%, 1.7-3.7 min B 90%, 3.71-5.5 min B 40%
Condition 2 (for measurement of Compound 1 and metabolites M5)
Mobile phase: A: 10 mM aqueous ammonium acetate, B: 0.1% formic acid/acetonitrile
Gradient condition: 0.0-3.0 min B 10% to 90%, 3.0-3.5 min B 90%, 3.51-5.0 min B 10%

Acetonitrile was added to plasma samples from mice and monkeys treated with Compound 1 to conduct deproteinization, and the resulting supernatant after centrifugation was used as the LC/MS/MS sample. In addition, standard solutions of synthesized Compound 1, Reference Example 18, Reference Example 9, and Reference Example 19 prepared in acetonitrile were added to blank plasma from mice and monkeys not treated with Compound 1 to conduct deproteinization, and the resulting supernatant after centrifugation was used as the sample for LC/MS/MS analysis.

The LC/MS/MS analysis results are shown in the table below. Under each set of conditions, the target ion was isolated in Q1 and subsequently fragmented, and in Q3, the compound-specific product ions were selected and detected.

**[Table 8]**

| Condition 1 | Compound 1 and synthetic | M2 and Reference | M4 and Reference |
|---|---|---|---|
| | standard | example 18 | example 9 |
| Q1 (*m*/*z*) | 416.34 | 449.22 | 321.16 |
| Q3 (*m*/*z*) | 348.21 | 100.96 | 140.95 |
| Retention time (min) | 3.0 | 2.8 | 2.6 |

**[Table 9]**

| Condition 2 | Compound 1 and synthetic standard | M5 and Reference example 19 |
|---|---|---|
| Q1 (*m*/*z*) | 416.34 | 366.24 |
| Q3 (*m*/*z*) | 348.21 | 141.50 |
| Retention time (min) | 2.7 | 2.7 |

As shown in the table above, it was confirmed that the retention times of the metabolites (M2, M4, and M5) present in plasma from mice and monkeys treated with Compound 1 were consistent with those of the synthesized Reference Examples 18, 9, and 19, respectively. Thus, the chemical structures of metabolite M2, metabolite M4, and metabolite M5 shown in Figure 21 were confirmed to be correct.

U-87 cells were cultured for 7 days in sphere medium in the presence of metabolites M2, M4, and M5, respectively. After culture, cellular ATP levels were quantified as an indicator of cell viability, and cell viability was calculated in the same manner as in Example 12 to compare cytotoxicity.

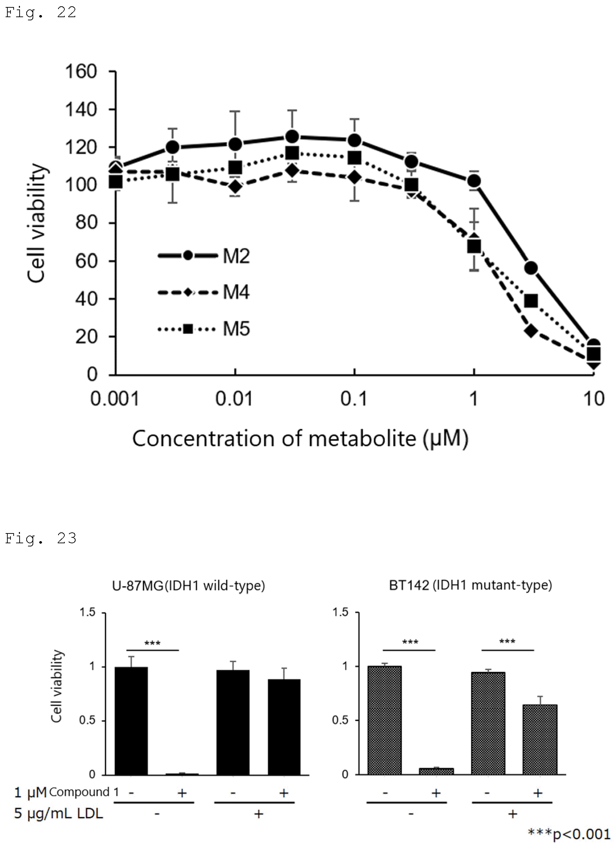

As shown in Figure 22, metabolites M2, M4, and M5 exhibited cytotoxicity under sphere culture conditions using U-87 cells. The calculated IC₅₀ values were 3.6 µM for M2, 1.6 µM for M4, and 2.1 µM for M5.

### Example 15: Cytotoxicity Assay under Sphere Culture Conditions Using Cell Lines Derived from Low-grade Glioma

U-87 cells (IDH1 wild-type) and BT142 cells derived from low-grade glioma (IDH1 mutant-type, R132H/-) were cultured for 7 days in LDL-supplemented sphere medium in the presence or absence of Compound 1 (1 µM). The LDL concentration was 5 µg/mL. After incubation, intracellular ATP levels were quantified as an indicator of cell viability, and cytotoxicity was compared between conditions.

As shown in Figure 23, even in the presence of LDL, Compound 1 exhibited marked cytotoxicity in the low-grade glioma-derived BT142 cell line.

As described in Example 12, in glioma tissues where the blood-brain barrier may be disrupted, intracellular LDL levels are likely elevated, potentially leading to reduced dependence on the endogenous cholesterol biosynthesis pathway. However, the results of Example 15 suggest that, in target diseases where IDH1 mutations are clinically observed, such as low-grade glioma, the impact of elevated LDL levels is mitigated even under high-LDL conditions, and Compound 1 retains sufficient cytotoxic activity. These findings indicate that low-grade glioma may represent a potential therapeutic indication for Compound 1.

### INDUSTRIAL APPLICABILITY

The pharmaceutical composition of the present disclosure comprises a dihydrochromene derivative or a pharmaceutically acceptable salt thereof, or a hydrate or solvate thereof, and can be used as a medicament for the treatment or prevention of cancer.

## Claims

1. A medicament for treating or preventing a cancer, comprising (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] (hereinafter, also referred to as "the present compound") or a pharmaceutically acceptable salt thereof, or a hydrate or solvate thereof as an active ingredient, which is orally administered to a subject.

2. The medicament of claim 1, wherein the active ingredient is (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] diphosphate, or a hydrate or solvate thereof.

3. The medicament of claim 1, wherein the active ingredient is (4'aR,10'bR)-8'-chloro-1-[2-(1H-imidazol-1-yl)ethyl]-5',5'-dimethyl-4'a,10'b-dihydro-2'H,4'H,5'H-spiro[piperidine-4,3'-pyrano[3,2-c][1]benzopyran] diphosphate (referred to as "Compound 1").

4. The medicament of any one of claims 1 to 3, which is orally administered to a subject once a day.

5. The medicament of any one of claims 1 to 3, which is orally administered to a subject twice a day.

6. The medicament of any one of claims 1 to 5, wherein the single dose of the active ingredient is an amount equivalent to 20 mg of the present compound.

7. The medicament of any one of claims 1 to 5, wherein the single dose of the active ingredient is an amount equivalent to 40 mg of the present compound.

8. The medicament of any one of claims 1 to 5, wherein the single dose of the active ingredient is an amount equivalent to 60 mg of the present compound.

9. The medicament of any one of claims 1 to 5, wherein the single dose of the active ingredient is an amount equivalent to 80 mg of the present compound.

10. The medicament of any one of claims 1 to 5, wherein the single dose of the active ingredient is an amount equivalent to 100 mg of the present compound.

11. The medicament of any one of claims 1 to 5, wherein the single dose of the active ingredient is an amount equivalent to 120 mg of the present compound.

12. The medicament of any one of claims 1 to 5, wherein the single dose of the active ingredient is an amount equivalent to 140 mg of the present compound.

13. The medicament of any one of claims 1 to 5, wherein the single dose of the active ingredient is an amount equivalent to 160 mg of the present compound.

14. The medicament of any one of claims 1 to 5, wherein the single dose of the active ingredient is an amount equivalent to 180 mg of the present compound.

15. The medicament of any one of claims 1 to 5, wherein the single dose of the active ingredient is an amount equivalent to 200 mg of the present compound.

16. The medicament of any one of claims 1 to 5, wherein the single dose of the active ingredient is an amount equivalent to 220 mg of the present compound.

17. The medicament of any one of claims 1 to 5, wherein the single dose of the active ingredient is an amount equivalent to 240 mg of the present compound.

18. The medicament of any one of claims 1 to 5, wherein the single dose of the active ingredient is an amount equivalent to 260 mg of the present compound.

19. The medicament of any one of claims 1 to 5, wherein the single dose of the active ingredient is an amount equivalent to 280 mg of the present compound.

20. The medicament of any one of claims 1 to 5, wherein the single dose of the active ingredient is an amount equivalent to 300 mg of the present compound.

21. The medicament of any one of claims 1 to 5, wherein the single dose of the active ingredient is an amount equivalent to 320 mg of the present compound.

22. The medicament of any one of claims 1 to 5, wherein the single dose of the active ingredient is an amount equivalent to 340 mg of the present compound.

23. The medicament of any one of claims 1 to 5, wherein the single dose of the active ingredient is an amount equivalent to 360 mg of the present compound.

24. The medicament of any one of claims 1 to 5, wherein the single dose of the active ingredient is an amount equivalent to 380 mg of the present compound.

25. The medicament of any one of claims 1 to 5, wherein the single dose of the active ingredient is an amount equivalent to 400 mg of the present compound.

26. The medicament of any one of claims 1 to 25, which is used in combination with a concomitant drug or a pharmaceutically acceptable salt thereof to treat a cancer, wherein the concomitant drug is at least one drug selected from an antitumor alkylating agent, an antitumor antimetabolite, an antitumor antibiotic, a plant-derived antitumor medicament, an antitumor platinum complex compound, an antitumor camptothecin derivative, an antitumor tyrosine kinase inhibitor, an antitumor serine/threonine kinase inhibitor, an antitumor phospholipid kinase inhibitor, an antitumor monoclonal antibody, interferon, a biological response modifier, a hormonal preparation, an angiogenic inhibitor, an immune checkpoint inhibitor, an epigenetics-associated molecular inhibitor, a protein post-translational modification inhibitor, a proteasome inhibitor, an enzyme inhibitor, a cholesterol absorption inhibitor, and other antitumor medicaments.

27. The medicament of any one of claims 1 to 25, which is used in combination with a concomitant drug or a pharmaceutically acceptable salt thereof to treat a cancer, wherein the concomitant drug is at least one selected from an antitumor alkylating agent, an antitumor monoclonal antibody, an immune checkpoint inhibitor, an enzyme inhibitor, a cholesterol absorption inhibitor, and other antitumor medicaments.

28. The medicament of claim 26 or 27, wherein the immune checkpoint inhibitor is an anti-PD-1 antibody or an anti-PD-L1 antibody.

29. The medicament of claim 28, wherein the anti-PD-1 antibody or the anti-PD-L1 antibody is pembrolizumab, nivolumab, atezolizumab, durvalumab, avelumab, cemiplimab, dostarlimab, toripalimab, or tislelizumab.

30. The medicament of claim 26 or 27, wherein the antitumor monoclonal antibody is bevacizumab or ipilimumab.

31. The medicament of claim 26 or 27, wherein the antitumor alkylating agent is temozolomide.

32. The medicament of claim 26 or 27, wherein the enzyme inhibitor is an IDH inhibitor.

33. The medicament of claim 32, wherein the IDH inhibitor is ivosidenib, vorasidenib, olutasidenib, or enasidenib.

34. The medicament of claim 26 or 27, wherein the enzyme inhibitor is an IDH1 inhibitor.

35. The medicament of claim 34, wherein the IDH1 inhibitor is ivosidenib, vorasidenib, or olutasidenib.

36. The medicament of claim 26 or 27, wherein the cholesterol absorption inhibitor is ezetimibe.

37. The medicament of any one of claims 1 to 25, which is used in combination with a concomitant drug or a pharmaceutically acceptable salt thereof to treat a cancer, wherein the concomitant drug is at least one selected from
(1) bevacizumab,
(2) ipilimumab,
(3) temozolomide,
(4) ivosidenib,
(5) vorasidenib, and
(6) ezetimibe.

38. The medicament of any one of claims 1 to 37, which is used in combination with a cell medicine.

39. The medicament of claim 38, wherein the cell medicine is chimeric antigen receptor T-cell (CAR-T cell).

40. The medicament of any one of claims 1 to 39, which is used in combination with radiotherapy.

41. The medicament of any one of claims 1 to 40, wherein the cancer is an anti-PD-1 antibody drug-resistant cancer.

42. The medicament of any one of claims 1 to 41, wherein the cancer is glioma.

43. The medicament of claim 42, wherein the glioma is malignant glioma.

44. The medicament of claim 43, wherein the malignant glioma is recurrent malignant glioma.

45. The medicament of any one of claims 42 to 44, wherein the glioma is diffuse glioma, pediatric-type diffuse low-grade glioma, pediatric-type diffuse high-grade glioma, circumscribed astrocytic glioma, glioneuronal and neuronal tumor, or ependymal tumor.

46. The medicament of claim 45, wherein the glioma is diffuse glioma.

47. The medicament of claim 45 or 46, wherein the diffuse glioma is astrocytoma, oligodendroglioma, or glioblastoma.

48. The medicament of any one of claims 45 to 47, wherein the diffuse glioma is glioblastoma.

49. The medicament of claim 47 or 48, wherein the glioblastoma is secondary glioblastoma.

50. The medicament of claim 45 or 46, wherein the diffuse glioma is astrocytoma or oligodendroglioma.

51. The medicament of claim 45 or 46, wherein the diffuse glioma is anaplastic oligodendroglioma or anaplastic astrocytoma.

52. The medicament of claim 45 or 46, wherein the diffuse glioma is pilocytic astrocytoma.

53. The medicament of claim 42, wherein the glioma is grade I or II based on the WHO 2016 classification of brain tumors.

54. The medicament of claim 42, wherein the glioma is grade II or III based on the WHO 2016 classification of brain tumors.

55. The medicament of claim 42, wherein the glioma is grade III or IV based on the WHO 2016 classification of brain tumors.

56. The medicament of claim 42, wherein the glioma is grade I to III based on the WHO 2016 classification of brain tumors.

57. The medicament of any one of claims 1 to 56, wherein the cancer is a cancer in which emopamil binding protein is highly expressed compared to that of healthy individuals.

58. The medicament of any one of claims 1 to 57, wherein the cancer is a cancer with IDH mutation.

59. The medicament of any one of claims 1 to 40, which is administered to a subject having IDH mutation.

60. The medicament of claim 59, wherein the subject having IDH mutation is determined through the following steps:
(1) detecting IDH mutation in cancer cells obtained from the subject, and
(2) determining the presence or absence of the IDH mutation detected in (1).
